# EUROPEAN PATENT APPLICATION

(11) **EP 4 082 523 A1**
(43) Date of publication of application: **02.11.2022**
(21) Application number: 20901480.2
(22) Date of filing: 16.12.2020
(51) Int. Cl.: A61K 8/96, A61Q 5/04, A45D 7/04, C02F 1/467, C02F 1/461

(54) **HAIR PERM FORMING METHOD USING ELECTROLYZED WATER**

(30) Priority: 17.12.2019 KR 20190169040
(71) Applicant: Konkuk University Industrial Cooperation Corp., Gwangjin-gu Seoul 05029 (KR)
(72) Inventor: KANG, Sang Mo, Seoul 04426 (KR); CHO, Young Jae, Seoul 01739 (KR)
(74) Representative: EP&C
(86) International application number: PCT/KR2020/018438
(87) International publication number: WO 2021/125783

(57) **Abstract**

A hair perm forming method using electrolyzed water multiple times or for a predetermined period of time or more of the present invention was revealed to have effects that cause very little damage to the hair to enable repeated treatments, enable perming of damaged hair and blonde hair compared with existing methods, show oxidative power higher than that of hydrogen peroxide, cause less hair damage, suppress dandruff bacteria, and significantly decrease the elution of dyes after perming. Therefore, the hair perm forming method using electrolyzed water multiple times or for a predetermined period of time or more of the present invention can be advantageously used as a method capable of effectively forming a perm without harm to the human body in the field of hair perm forming. In addition, the washing of dyed and permed hair, dyed hair, and bleached hair with electrolyzed water, compared with washing with distilled water, has advantages in that the tensile strength of hair is maintained and the dye loss from the dyed hair is less.

## Description

### Technical Field

The present invention relates to a method of forming a hair perm and, more particularly, to a method of forming a hair perm using electrolyzed water.

### Background Art

Two types of agents are used in the current perm process. A reduction agent, which is a first agent used in a perm process, includes a component having reducing power such as thioglycolic acid or cysteine. These components break down disulfide bonds by reducing cystine in hair. In this case, cysteine mainly breaks down disulfide bond of non-helical intermediate filament and thioglycolic acid breaks down disulfide bond between intermediate filament and matrix protein, or matrix proteins. Due to these properties, thioglycolic acid has a higher perm efficiency than cysteine, but causes slightly more damage to the hair.

When the shape of hair is physically fixed and treated with an oxidation agent, which is a second agent used in a perm process, the oxidation agent fixes the shape of hair by oxidizing the reduced cysteine to form a disulfide bond. Th oxidation agent includes a component having oxidizing power such as sodium bromate or hydrogen peroxide. In general, hydrogen peroxide has a better perm efficiency than sodium bromate.

Hydrogen peroxide used as an oxidation agent causes fatal damage to hair. As the treatment time of hydrogen peroxide increases, hair damage increases and it has been reported that the ratio of cysteic acid, which is known as an indicator of hair damage, increases. Due to such irreversible damage, damaged hair is difficult to perm again.

In addition, hydrogen peroxide causes an inflammatory reaction in the skin and causes skin erythema, etc. It also was reported that hydrogen peroxide causes DNA damage in an in vitro experiment. In particular, it is known that the molecule of hydrogen peroxide is small and non-polar, so it can easily pass through the cell membrane and cause greater damage. Further, in animal experiments using mice, it has been reported that the bleaching agent using hydrogen peroxide causes skin swelling and chemical burns on the skin.

Similarly, bromate, which is used as an oxidation agent, has also been found to be harmful to the human body. Long-term exposure to bromate causes dizziness and hearing damage. The International Agency for Research on Cancer (IARC) designates potassium bromate as a Group 2B carcinogen.

### Related Art Document

### Patent Document

Korean patent application publication No. 10-2018-0045158 (May 4, 2018)
Korean patent application publication No. 10-2012-0095321 (August 28, 2012)

### Disclosure

### Technical Problem

Hydrogen peroxide and bromate used in permanent waves are harmful to the human body and damage the hair, so they cannot form a complete shape of perm during repeated treatments. Therefore, while researching a method for replacing harmful hydrogen peroxide and bromate during perm treatment, the inventors of the present invention completed the present invention based on the idea that a phenomenon of the failure to form a complete shape of perm during repeated treatments is due to peroxidative and oxidative damage of cystine, which plays a major role in maintaining the structure of hair, and electrolyzed hydrogen water and electrolyzed water having limited oxidizing power are used to reduce such damage. In addition, another objective of the present invention is to provide a method of forming a permanent wave that can achieve a permanent wave for chemically weak hair such as blonde hair.

In addition, another objective of the present invention is to provide a composition for washing hair, including electrolyzed water.

In addition, another objective of the present invention is to provide a method of washing hair, including the step of treating the hair with electrolyzed water.

### Technical Solution

According to one aspect of the present invention, there is provided a method of forming a hair perm, the method comprising: (a) treating the hair with a reduction agent and then fixing the hair; and (b) treating the fixed hair with electrolyzed water twice or more.

In one embodiment, the hair in step (a) may be one or more hairs selected from the group consisting of natural black hair, blonde hair, bleached hair, curly hair, and brown hair.

In one embodiment, the electrolyzed water of step (b) may be electrolyzed hydrogen water or electrolyzed water, wherein the electrolyzed hydrogen water may be prepared in a hydrogen water generator equipped with a cation exchange membrane, and the electrolyzed water may be prepared with a process of electrolyzing water without separating an anode and a cathode.

In one embodiment, the electrolyzed water in step (b) may be prepared by an electrolyzed water generator with an output of 11W or more and may be used within 30 minutes from being prepared.

In one embodiment, the electrolyzed water in step (b) may have a temperature of 10°C to 90°C and a pH of 0.7 to 11.5.

In one embodiment, the perm may be a permanent wave or a straight perm.

According to another aspect of the present invention, provided is electrolyzed water for forming a hair perm.

In one embodiment, the electrolyzed water may be electrolyzed hydrogen water or electrolyzed water, wherein the electrolyzed hydrogen water may be prepared in a hydrogen water generator equipped with a cation exchange membrane, and the electrolyzed water may be prepared with a process of electrolyzing water without separating an anode and a cathode.

In one embodiment, the electrolyzed water may be prepared by an electrolyzed water generator with an output of 11W or more.

In one embodiment, the electrolyzed water may have a temperature of 10°C to 90°C and a pH of 0.7 to 11.5.

In addition, provided is a composition for washing hair, including electrolyzed water.

In addition, provided is a method of washing hair, including the step of treating the hair with electrolyzed water.

In one embodiment of the present invention, the electrolyzed water may be electrolyzed water or hydrogen water.

In one embodiment of the present invention, the hair may be dye/perm-treated hair or dye-treated hair.

### Advantageous Effects

According to a method of forming a hair perm using electrolyzed water of the present invention, wherein the method uses the electrolyzed water several times or for a specific time or longer, the method has been found to have the effects enabling to repeat treatments due to very little damage to hair, to perm damaged hair and blonde hair compared to the existing method, to have an oxidizing power greater than hydrogen peroxide, to cause less damage to hair, to suppress dandruff, and to significantly reduce dye elution after the perm. Therefore, the method of forming a hair perm, using electrolyzed water of the present invention several times or for a specific time or longer, can be usefully used as a method of effectively form a hair perm without harm to the human body in the field of forming a hair perm. In addition, in the case of washing dying/perm-treated hair, dyed hair, and bleached hair with electrolyzed water, compared to washing with distilled water, it has the advantage that the tensile strength of the hair is maintained and there is less loss of dye from the dyed hair.

### Description of Drawings

FIG. 1 is a graph illustrating the amount of cystine generated by reacting with cysteine after leaving hydrogen water (24W) and electrolyzed water (27W) prepared by generators of different outputs for different times;
FIG. 2 is a photograph showing the wave efficiency of natural black hair according to the number of treatments with hydrogen water as a second agent, during the perm treatment;
FIG. 3A is a photograph showing a change in hair (blonde) length when washing perm hair according to the number of treatments with hydrogen water as a second agent;
FIG. 3B is a wave efficiency (bleached hair) according to the number of treatments with electrolyzed water as a second agent during softening perm treatment;
FIG. 3C is a photograph showing the wave efficiency of brown hair according to the treatment with hydrogen peroxide as a second agent and hydrogen water 10 times during the perm treatment;
FIG. 3D is a photograph showing a change in hair length when using 25°C electrolyzed water as a second agent of perm;
FIG. 3E is a photograph showing a change in hair length when using 90°C electrolyzed water as a second agent of perm;
FIG. 4 is a photograph showing the wave efficiency in blonde hair when treated with hydrogen water cold perm generated by hydrogen water generators having different outputs;
FIG. 5 is a photograph showing the wave efficiency (natural black hair) when treated with hydrogen water cold perm generated by hydrogen water generators having different outputs;
FIG. 6 illustrates a comparison of wave efficiencies in blond hair during cold perm treatment with electrolyzed water and hydrogen water;
FIG. 7 illustrates a comparison of wave efficiencies in natural black hair during cold perm treatment with electrolyzed water and hydrogen water;
FIG. 8 illustrates a change in hair length (natural black hair) according to washing of cold perm-treated perm hair with pH 4-10 hydrogen water;
FIG. 9 illustrates a change in hair length (natural black hair) according to washing of cold perm-treated perm hair with pH 1-4 and pH 0.7 hydrogen water;
FIG. 10 illustrates a change in hair length (natural black hair) according to washing of cold perm-treated perm hair with pH 10-12 hydrogen water;
FIG. 11 illustrates a change in hair length (natural black hair) according to washing of cold perm-treated perm hair with pH 11.25-12.25 hydrogen water.
FIG. 12 illustrates a change in hair length (natural black hair) according to washing of cold perm-treated perm hair with pH 0.7, pH 1, and pH 7 electrolyzed water.
FIG. 13 illustrates a change in hair length (natural black hair) according to washing of cold perm-treated perm hair with pH 10-12 electrolyzed water.
FIG. 14 illustrates a change in hair length (natural black hair) according to washing of cold perm-treated perm hair with hydrogen water at different temperatures [Lane 1: 20°C; Lane 2: 40°C; Lane 3: 60°C; Lane 4: 80°C; Lane 5: 90°C].
FIG. 15 illustrates a change in hair length (natural black hair) according to washing of cold perm-treated perm hair with hydrogen water at different temperatures.
FIG. 16 illustrates a change in hair length (natural black hair) according to washing of cold perm-treated perm hair with electrolyzed water at different temperatures.
FIG. 17 illustrates a result of straight perm treatment of curly hair [1-1: hydrogen peroxide water treatment; 1-2: hydrogen water treatment].

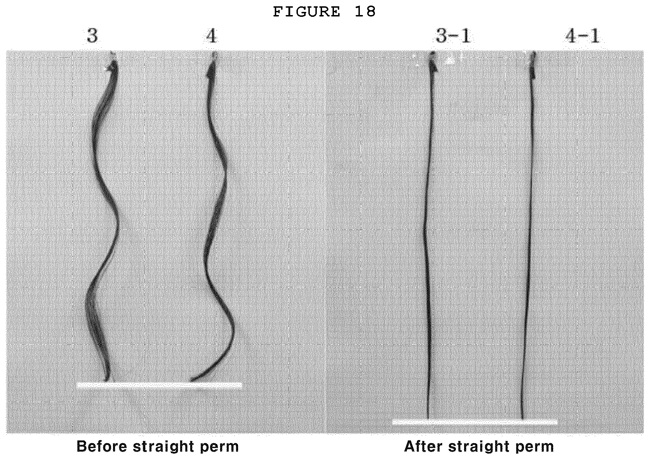

FIG. 18 illustrates a result of straight perm treatment of curly hair [3-1: hydrogen peroxide water treatment; 4-1: hydrogen water treatment].
FIG. 19 is an ATR-FTIR spectrum (1,300^{∼}1,000cm⁻¹) of perm hair using hydrogen water and electrolyzed water [Color lines: green, control; red, hydrogen peroxide-based perm hair; purple, electrolytic hydrogen water-based perm hair; blue, electrolyzed water-based perm hair].
FIG. 20 is a photograph of a human hair wig (before perm).
FIG. 21 is a photograph of a human hair wig (one wash after perming) .
FIG. 22 is a photograph of a human hair wig (15 washes after perming).
FIG. 23 is a photograph of a human hair wig (30 washes after perming).
FIG. 24 illustrates the sterilization effect of *Malassezia furfur* by electrolyzed water.
FIG. 25 illustrates the appearance of hair when treated with hydrogen peroxide as a second agent, electrolyzed water, or hydrogen water during perm treatment;
FIG. 26 is a length measurement and photographs taken after washing to measure perm efficiency using hydrogen peroxide, electrolyzed hydrogen water, and electrolyzed water (line 1: washed with distilled water, line 2: first time with hydrogen water and others with distilled water, line 3: hydrogen water, line 4: first time with electrolyzed water and others with distilled water, and line 5: electrolyzed water);
FIG. 27 illustrates the results of washing with distilled water, hydrogen water, and electrolyzed water for dark brown dyed hair;
FIG. 28 illustrates the results of washing with distilled water, hydrogen water, and electrolyzed water for red dyed hair;
FIG. 29 illustrates the results of washing with distilled water, hydrogen water, and electrolyzed water for blue dyed hair; and
FIG. 30 is a result of a comparative experiment on the speed of making cysteine into cystine when using hydrogen water generator or an electrolyzed water generator.

### Best Mode

The present invention provides a method of forming a hair perm including: (a) treating the hair with a reduction agent and then fixing the hair; and (b) treating the fixed hair with electrolyzed water twice or more.

In the present invention, electrolyzed water is used in order to overcome the toxicity or adverse effects of hydrogen peroxide or bromate used as an oxidation agent in hair perm treatment. Typically, it is known that H₂ and O₂ are generated during electrolysis process, but·OH, O₃, H₂O₂, ·O₂-, ·H are also generated during electrolysis process of water. At the same time, when chlorine is included in electrolyte, oxidization substances such as HOCl and OCl⁻, etc. are generated. In the case of electrolyzed water prepared in this way, acidic electrolyzed water generated at the anode and basic electrolyzed water generated at the cathode are generated, and both do not generate harmful residues and thus, are used for food sterilization. When these two electrolyzed waters are mixed, they are generally neutralized and should be the same as those of general water component. However, they have a weak reducing power compared to basic electrolyzed water and acidic electrolyzed water but at the same time contain oxidization substances such as HOCL, which occupies a large proportion in the oxidizing power of electrolyzed water.

Recently, after research results showing that hydrogen gas and radicals exhibit antioxidant activity, hydrogen water generators through electrolysis have been sold. When water is electrolyzed, hydrogen ions and oxygen molecules are generated at the anode and hydroxide ions and hydrogen molecules are generated at the cathode. When they are separated, the pH of the anode increases. In this case, when separated by Nafion, which is a cation exchange membrane, hydrogen ions generated at the anode move to the cathode to neutralize hydroxide ions. In cathode, water of a large amount of hydrogen molecules dissolved and a pH of close to neutral can be obtained, and such water is referred to as "hydrogen water" or "electrolyzed hydrogen water". In the present invention, hydrogen water was prepared and experimented using a hydrogen water generator equipped with Nafion, which is a cation exchange membrane.

The term "electrolyzed water" refers to water in which the generated hydrogen ions and hydroxide ions are neutralized by not separating the cathode and anode, and as a result, are neutral and both oxygen and hydrogen molecules have high concentrations.

Unlike HOCL, which lasts more than 30 days after generation, ·OH, O₃, H₂O₂, and ·O₂-, which are active oxygen generated together during the electrolysis process, are prepared in small amounts and chemically unstable to prepare other radicals or disappear by reacting with other molecules. However, according to the preliminary experiment, the cystine generating power of electrolyzed water disappears within 1 hour (mostly disappear within 25 or 30 minutes), and even with an electrolyte that does not include chlorine such as NaH₂PO₄, the same perm forming power and cystine generating power are exhibited as when NaCl is added. Through it, it is estimated that the perm is formed by radicals.

Although a high concentration of hydrogen peroxide can be generated through electrolysis, but when a catalyst is not used as in the present invention, the generation rate is very low. For example, as a result of electrolysis under condition of 0.8A and a hydrogen gas flow rate of 1.2×10⁻⁷m³·s⁻¹ for 3 hours, 5.88×10-⁴mol is generated. When the corresponding value is applied to the experiment of the present invention, it is 2.45×10⁻⁴ mol, which is too low compared to the 2% hydrogen peroxide solution used in the existing perm agent. In addition, in the case of hydrogen peroxide, it remains after washing to cause additional delayed oxidation and in the case of electrolyzed water, no harmful residues remain and there is no delayed oxidation because all substances are finally returned to water.

The method of forming a perm of the present invention includes a step of fixing the hair after treating the hair with a reduction agent (i.e., step (a)). Step (a) is a step of decomposing a disulfide bond by reducing the cystine of the hair. In step (a), after treating the hair with a reduction agent, the hair may be fixed with or without reduction agent removed. In addition, the hair may be applied without limitation as long as the hair is perm-formable (human hair or wig hair), for example, it may be one or more hairs selected from the group consisting of natural black hair, blonde hair, bleached hair, curly hair, and brown hair, but is not limited thereto.

A method of forming a hair perm of the present invention includes a step of treating the fixed hair with electrolyzed water twice or more (i.e., step (b)). Step (b) is a step of physically fixing a shape of hair and treating it with an oxidation agent, which fixes the shape of hair by oxidizing the reduced cysteine to form a disulfide bond. In step (b), the electrolyzed water may be electrolyzed hydrogen water or electrolyzed water, the electrolyzed hydrogen water may be prepared in a hydrogen water generator equipped with a cation exchange membrane, and the electrolyzed water may be prepared by a process of electrolyzing water without separating an anode and a cathode.

In one embodiment, the electrolyzed water in step (b) may be prepared by an electrolyzed water generator with an output of 11W or more, and preferably, 15W or more, and more preferably, 20W or more.

In one embodiment, the electrolyzed water in step (b) may be used within 30 minutes from being prepared.

In one embodiment, the electrolyzed water in step (b) may have a temperature of 10°C to 90°C, and preferably, 10°C to 40°C. When electrolyzed water of about 25°C is used, the treatment time may be about 55 seconds or longer, preferably 1 minute or more, and when electrolyzed water of about 90°C is used, the treatment time is about 40 seconds or more, preferably 45 seconds or more.

In one embodiment, the electrolyzed water in step (b) may have a pH of 0.7 to 11.5.

In one embodiment, the perm may be a permanent wave or a straight perm.

The present invention provides electrolyzed water for forming a hair perm.

In one embodiment, the electrolyzed water of step (b) may be electrolyzed hydrogen water or electrolyzed water wherein the electrolyzed hydrogen water may be prepared in a hydrogen water generator equipped with a cation exchange membrane, and the electrolyzed water may be prepared by a process of electrolyzing water without separating an anode and a cathode.

In one embodiment, the electrolyzed water may be prepared by an electrolyzed water generator with an output of 11W or more, preferably 15W or more, and more preferably, 20W or more.

In one embodiment, the electrolyzed water may have a temperature of 10°C to 90°C, and preferably, 10°C to 40°C.

In one embodiment, the electrolyzed water may have a pH 0.7 to 11.5.

In another aspect, the present invention provides a composition for washing hair, including electrolyzed water.

In another aspect, the present invention provides a method of washing hair, including the step of treating the hair with electrolyzed water.

In one embodiment of the present invention, the electrolyzed water may be electrolyzed water or hydrogen water.

In one embodiment of the present invention, the hair may be dying/perm-treated hair or dye-treated hair.

The wave efficiency may be maintained better by washing the perm hair with electrolyzed water.

In one embodiment of the present invention, the composition may be used to prevent loss of dye in the hair.

In one embodiment of the present invention, the washing method may be a method of washing hair by dye-treating or dying/perm treating the hair, and then applying electrolyzed water to the hair.

In one embodiment of the present, it was confirmed that the tensile strength of the hair was maintained stronger than that of washing with distilled water and that electrolyzed water is more effective than hydrogen water.

In one embodiment of the present invention, when washing dying/perm-treated hair or dyed hair, using electrolyzed water, it was confirmed that loss of dye was less than that of washing with distilled water, and electrolyzed water was more effective than hydrogen water. The perm hair can be washed with electrolyzed water to maintain better wave efficiency.

Unless contradictory each other, the description of hydrogen water, electrolyzed water, and preparing method thereof, etc. related to the above-described method of forming the hair may be applied to the hair washing composition and hair washing method.

### Mode for Invention

Hereinafter, the present invention will be described in more detail with reference to the embodiments. The following examples only illustrate the present invention, and the present invention is not limited by the examples.

### <Examples>

### 1. Measurement of Cystine Generation Capacity in Hydrogen Water and Electrolyzed water

Cysteine is unstable in air and has the property of combining with each other to form cystine (Hirs, C. W. (1967) . Performic acid oxidation. In Methods in enzymology (Vol. 11, pp. 197-199). Academic Press.). In addition, cystine precipitates insoluble in water. Through such properties, the amount of cystine prepared was measured by adding electrolyzed hydrogen water and electrolyzed water with different open times to cysteine. For preparation of the used electrolyzed hydrogen water and electrolyzed water, a 005% NaCl solution prepared by adding NaCl to distilled water was used. 400 ml of raw material water was put into a hydrogen water generator (Grentech, Korea) with an output of 24W (12V, 2A) and hydrogen water generation process was performed for 20 minutes to obtain hydrogen water. The raw material water was put into an electrolyzed water generation (Ecowell, Korea) with an output of 27W (9V, 3A) and electrolyzed water generation process was performed for 20 minutes to obtain electrolyzed water, which was used as electrolyzed water.

After putting 0.75g of cysteine into each of seven 15 ml test tubes, electrolyzed hydrogen water and electrolyzed water were left for 5 minutes, 10 minutes, 15 minutes, 20 minutes, 25 minutes, and 30 minutes, respectively, immediately after the generation of electrolyzed hydrogen water and electrolyzed water, of which 15 ml each was injected into 6 test tubes containing cysteine and reacted for 30 minutes, respectively. Since the cysteine dissolved in the cysteine solution is oxidized even at oxygen concentration in the air, the supernatant containing unreacted cysteine was removed by centrifugation (2000 x G, 5 minutes) after the reaction time was elapsed to prevent further reactions over time.

After the precipitated cystine was suspended by adding 3 ml of distilled water, turbidity was measured at 660 nm, and the amount of cystine preparation was calculated using a standard. For experimental control group, 0.05% NaCl solution generated by adding NaCl to distilled water was boiled, cooled, and injected into the other cysteine-containing test tube and reacted for 30 minutes. The above experiment was repeated three times and the mean and standard error were calculated through IBM SPSS statistics for Windows, version 20.0 (IBM, USA).

The pH of electrolyzed hydrogen water and electrolyzed water prepared by electrolysis before the experiment was 6.8, and there was no difference before and after electrolysis.

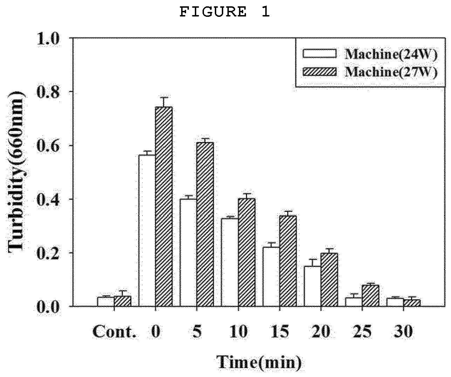

The turbidity of the precipitate generated by adding electrolyzed hydrogen water and electrolyzed water to cysteine is illustrated in FIG. 1. Immediately after the generation of electrolyzed hydrogen water and electrolyzed water, and after leaving each differently at 5 minute-intervals from immediately after generation, the resulting precipitate was measured by adding it to cysteine. As a result of measuring the concentration of the product in turbidity, the concentrations of the hydrogen water (24W apparatus) were 0.563, 0.399, 0.323, 0.206, 0.148, 0.030, and 0.029. Those of electrolyzed water (27 W apparatus) were 0.751, 0.602, 0.402, 0.328, 0.199, 0.069, and 0.028. The experimental control group showed 0.031 and 0.039 when reacted for 30 minutes, which was almost the same as the value obtained by allowing the electrolyzed hydrogen water generated by the 24W apparatus that was left for 25 minutes, injected, and reacted for 30 minutes. In addition, in case of the electrolyzed water 27W apparatus, the value was about the same as the value that was left for 30 minutes, injected, and reacted for 30 minutes. As a result of adding electrolyzed hydrogen water to 0.75g of cysteine in this way, the resulting precipitate was calculated as weight of 0.0103g, 0.0073g. 0.0059g, 0.0038g, 0.0027g, 0.0005g, and 0.0005g, respectively, and the precipitate generated by adding electrolyzed water was calculated as weight of 0.0137g, 0.0110g. 0.0073g, 0.0060g, 0.0036g, 0.0013g, and 0.0005g, respectively. The experimental control groups were 0.0006g and 0.0007g. As such, it was found that the ability to promote cystine conversion from cysteine in electrolyzed hydrogen water disappears after 25 minutes. It was found that the ability to promote cystine conversion from cysteine in electrolyzed water disappears after 30 minutes. Therefore, it is desirable for wave efficiency to treat electrolyzed hydrogen water and electrolyzed water immediately after generation during perm treatment.

### 2. Comparison of Effects according to Repeated Perm Treatments of Electrolyzed Hydrogen Water as Second Agent and Temperature

### Natural Black Hair

Tap water was used as the raw material water used to compare the wave forming power of electrolyzed hydrogen water. For reference, in the perm experiment, the difference between the 0.05% NaCl solution generate by adding NaCl to distilled water and tap water was insignificant. A hydrogen water obtained through a hydrogen water generation process for 20 minutes in which 400 ml of 25°C tap water was put into a hydrogen water generator (Grentech, Korea) with an output of 24W (12V, 2A) was used in the experiment. Perm process was performed at normal room temperature (cold perm). In perm treatment, commercially available Hair 119 (Eson Chemical, Korea) was used as a first agent, and the main ingredients are cysteine HCL and cysteamine HCL. Electrolyzed hydrogen water was used as a second agent.

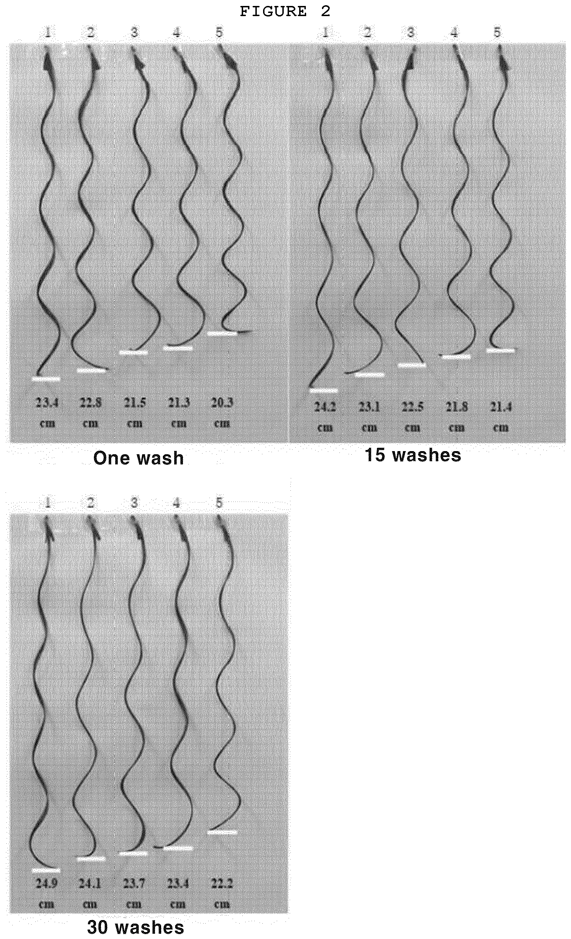

About 50 strands of natural black hair were fixed and cut to 27 cm in length and used in the experiment. The perm process was performed as follows. The natural black hair was fixed and washed according to the above standards. After washing, the dried hair bundle was reduced by treatment with the first agent (Hair 119 Clinic SuBoon Perm, Eson Chemical, Korea) at 37°C for 25 minutes. After the reduction process, the first agent was removed by washing with distilled water to increase the reproducibility of the wave efficiency. The washed hair was wound and fixed on a rod with a diameter of 8 mm. Electrolyzed hydrogen water was applied to the hair fixed on the rod at 2-minute intervals 1, 2, 4, 7, and 10 times to reoxidize. When repeatedly applied, it was treated with a pipette at a rate of 5 ml per time. After the application was finished, the rod was removed. In order to measure the perm efficiency, the length was measured, and photos was take after washing (FIG. 2). Table 1 below shows the results of change in the length of hair (natural black hair) during washing of the perm hair according to the number of times of treatment with the second hydrogen water during perm treatment.

**Table 1**

| Lane | 1 | 2 | 3 | 4 | 5 |
|---|---|---|---|---|---|
| Number of treatments of hydrogen water | 1 | 2 | 4 | 7 | 10 |
| One wash | 23.4 cm | 22.8 cm | 21.5 cm | 21.3 cm | 20.3 cm |
| 15 washes | 24.2 cm | 23.1 cm | 22.5 cm | 21.8 cm | 21.4 cm |
| 30 washes | 24.9 cm | 24.1 cm | 23.7 cm | 23.4 cm | 22.2 cm |

Table 1 below shows that the wave was maintained from 1 treatment to 30 washes of hydrogen water, but the wave appeared well in proportion to the number of treatments (FIG. 2). Therefore, it may be seen that the hydrogen water used as the second agent improves the wave as the number of treatment increases.

### 2) Blonde Hair

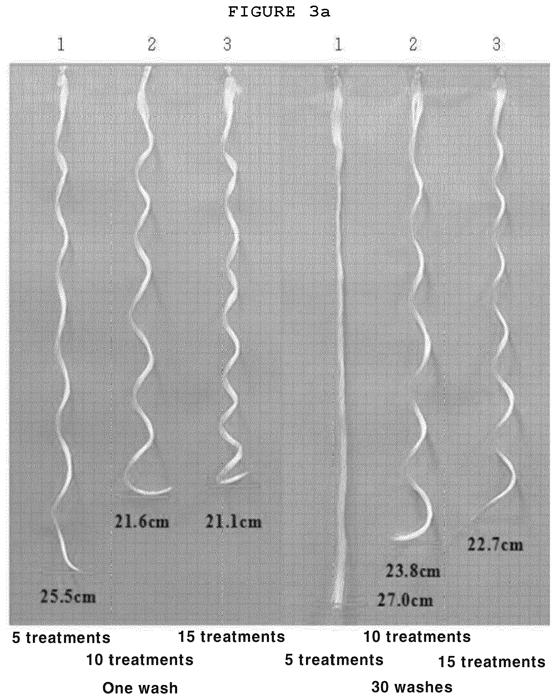

The blonde hair used in the experiment was a hair that had not been pretreatment (Moresoo, UK), and the hair used for perm treatment was divided into 27 cm long and 0.2g each and fixed. According to the method used in a general beauty salon using a rod, acetylcysteine (Calon Cystein, Iljin Cosmetic, Korea) treatment as a reduction agent, intermediate washing, setting, oxidation agent treatment, and measurement after washing were performed in the order. Blonde hair was treated with acetylcysteine as a reduction agent and left at 37°C for 5 minutes. In order to increase the reproducibility of wave efficiency, the hair was washed with distilled water to remove the reduction agent and then wound on a rod with a diameter of 8 mm in a spiral manner. Hydrogen water was treated 5, 10, and 15 times at 2-minute intervals. When repeatedly applied, it was treated with a pipette at a rate of 5 ml per time. Then the hair was separated. After washing them once, the length of perm hair was measured. After that, the perm efficiency was confirmed when repeatedly washed 30 times, once a day (FIG. 3A). Natural Shampoo (Repit, Korea) was used as a cleaning agent.

A method of preparing hydrogen water is as follows. After adding NaCl to distilled water to prepare a 0.05% NaCl solution, salted distilled water was boiled for about 5 minutes to remove dissolved oxygen as much as possible, and then quenched to 25°C (water cooling). The 400 ml prepared in this way was put into a hydrogen water generator (Grentech, Korea) with an output of 24W (12A, 2A) and hydrogen water generation process was performed for 20 minutes.

As a result of the experiment as descried above, the sample treated with hydrogen water 5 times had a good wave in one wash, but the wave disappeared after 30 washes. However, the samples treated with hydrogen water 10 times and 15 times had good wave durability even after 30 washes, and 15 treatments were the best. Therefore, wave durability was proportional to the number of hydrogen water treatments.

In the case of 5 times of hydrogen water treatment (lane 1) in the one-time washing samples after perm treatment, the wave was slightly longer than that of hydrogen water treatment 10 times (lane 2) and 15 times treatment (lane 3). When washing 30 times, the wave disappeared in the case of 5 times of hydrogen water treatment (lane 1), and the wave was maintained well in the case of 10 times of hydrogen water treatment (lane 2), and 15 times of hydrogen water treatment (lane 3) was the best. Therefore, even in the case of blonde hair, which is difficult to perm, it can be seen that the wave can be maintained for at least two months, assuming that it is washed once every 2 days with 10 treatments of hydrogen water.

### 3) Bleached Hair

Commercially available Hair 119 (Eson Chemical, Korea) was used as the first agent of a perm agent, and the main ingredients are cysteine HCL and cysteamine HCL. Electrolyzed hydrogen water was used as the second agent. About 50 strands of hair were fixed and cut to 27 cm in length and used in the experiment. The electrolyzed hydrogen water used as the second agent was prepared as follows. The electrolyte was dissolved in 400 ml of distilled water so that the NaCl concentration of the electrolyte was 0.05% (w/v) . 400 ml of raw material water was put into a hydrogen water generator (Grentech, Korea) and hydrogen water generation process was performed for 20 minutes. The bleached hair used in the experiment was prepared by mixing a natural black hair with a bleach developer (Koleston Perfect Creme Developer 6%, Wella, Germany) and bleach powder (Blondor Multi Blonde, Wella, Germany) at a ratio of 2:1 for 20 minutes. After that, the bleaching agent was removed by washing. This process was repeated three time to make and use bleached hair.

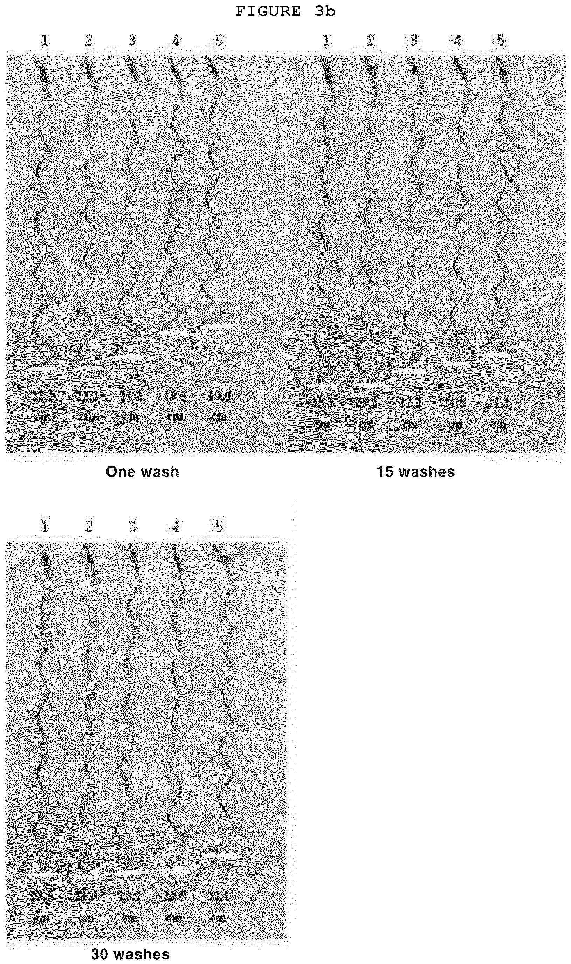

All perm experiments were performed at 27°C. First, considering the degree of damage to the hair, the first treatment time was 15 minutes for bleached hair to a level where the hair did not melt. In order to increase the reproducibility of the wave efficiency, the first agent of the hair was removed through intermediate washing and then wound and fixed on a rod with a diameter of 10 mm heated to 60°C. Hydrogen water was applied to the fixed hair once every two minutes 1, 2, 4, 7, and 10 times. When repeatedly applied, it was treated with a pipette at a rate of 5 ml per time. After the application was finished, the rod was removed to measure the perm efficiency of each hair and take pictures (FIG. 3B). Overall, the wave showed better results than the natural black hair of FIG. 2.

### 4) Brown Hair

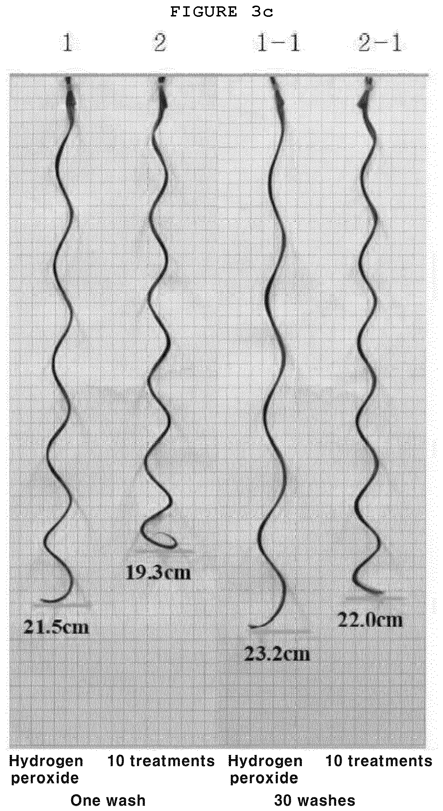

The brown hair used in the experiment was a hair that had not been pretreatment (Moresoo, UK), and the hair used for perm treatment was divided into 27 cm long and 0.2g each and fixed. According to the method used in a general beauty salon using a rod, cysteine treatment as a reduction agent, intermediate washing, setting, oxidation agent treatment, and measurement after washing were performed in the order. Brown hair was treated with a reduction agent (Hair 119 Clinic SuBoon Perm, Eson Chemical, Korea) and left at 37°C for 5 minutes. In order to increase the reproducibility of wave efficiency, the hair was washed with distilled water to remove the reduction agent and then wound on a rod with a diameter of 8 mm in a spiral manner. Hydrogen water was applied 10 times at 2-minute intervals, and as a control group, hydrogen peroxide (Hair 119 Clinic SuBoon Perm, Eson Chemical, Korea) with better perm efficiency than sodium bromate was applied and left for 10 minutes. Then the hair was separated. After washing them once, the length of perm hair was measured. After that, the perm hair length was measured when repeatedly washed 30 times, once a day (FIG. 3C). Natural Shampoo (Repit, Korea) was used as a cleaning agent.

A method of preparing hydrogen water is as follows. After adding NaCl to distilled water to prepare a 0.05% NaCl solution, salted distilled water was boiled for about 5 minutes to remove dissolved oxygen as much as possible, and then quenched to 25°C (water cooling). The 400 ml prepared in this way was put into a hydrogen water generator (Grentech, Korea) with an output of 24W (12A, 2A) and hydrogen water generation process was performed for 20 minutes.

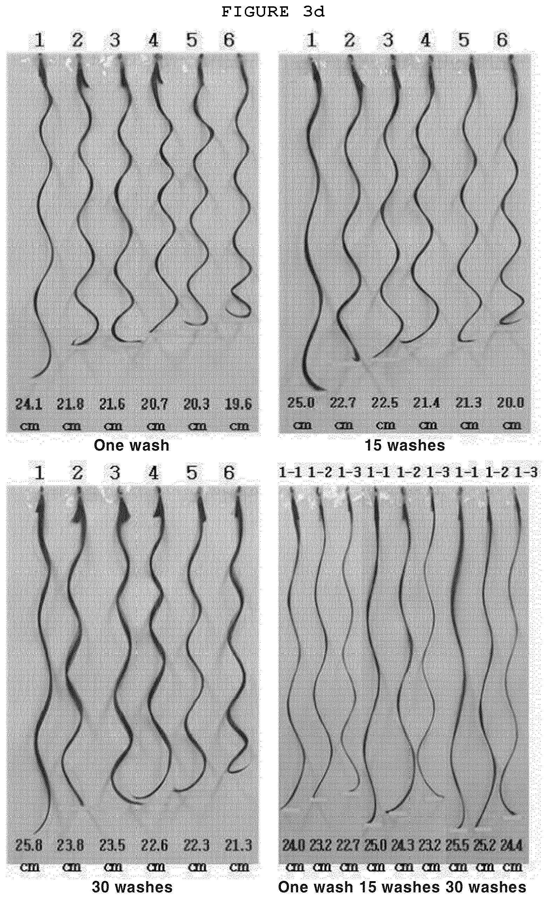

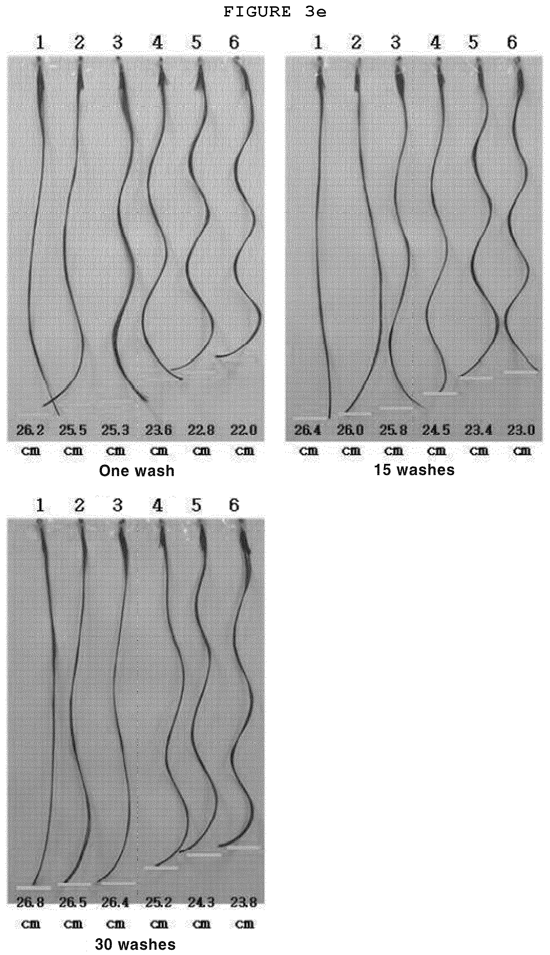

The results of the experiment as described above are shown in FIG. 3C. As shown in FIG. 3, compared to the case where hydrogen peroxide was treated and washed once (lane 1) and washed 30 times (lane 1-1), the case where hydrogen peroxide was treated 10 times (lane 2) and washed 30 times (lane 2-1) showed significantly better wave efficiency. In other words, in the case of hydrogen peroxide treatment, sagging was seen in 30 washes, but in the case of hydrogen water treatment, it was hardly seen. Therefore, when washing hair once every two days, the perm treatment of brown hair appears to be maintained for more than 2 months, indicating that commercialization is sufficient.

### 5) Use of 25°C Electrolyzed Water as Second Agent of Perm

Tap water was used as the raw material water used to compare the wave forming power of electrolyzed hydrogen water. 400 ml of 25°C tap water was put into an electrolyzed water generator with an output of 27W (9V, 3A) (Ecowell, Korea) and electrolyzed water generation process was performed for 20 minutes to obtain an electrolyzed water, which was used in the experiment. The perm process was performed at normal room temperature (cold perm) . In perm treatment, commercially available Hair 119 (Eson Chemical, Korea) was used as the first agent, and the main ingredients are cysteine HCL and cysteamine HCL. Electrolyzed hydrogen water was used as the second agent.

About 50 strands of natural black hair were fixed and cut to 27 cm in length and used in the experiment. The perm process was performed as follows. The natural black hair was fixed and washed according to the above standards. After washing, the dried hair bundle was reduced by treatment with the first agent (Hair 119 Clinic SuBoon Perm, Eson Chemical, Korea) at 37°C for 25 minutes. After the reduction process, the first agent was removed by washing with distilled water to increase the reproducibility of the wave efficiency. The washed hair was wound and fixed on a rod with a diameter of 8 mm. Electrolyzed water was put into a sprayer and applied while continuous spraying to the hair fixed on the rod at a spray speed of 10 ml/min to reoxidize. After the application was finished, the rod was removed. In order to measure the perm efficiency, the length was measured and photos were taken after washing (FIG. 3D).

The numbers shown in FIG. 3D are results of a change in length of hair (natural black hair) during washing of the perm hair according to the second electrolyzed water treatment during perm treatment [Lane 1: 30 sec. treatment; Lane 2: 1 min. treatment; Lane 3: 2 min. treatment; Lane 4: 3 min. treatment; Lane 5: 4 min. treatment; Lane 6: 5 min. treatment, Lane 1-1: 45 sec. treatment; Lane 1-2: 50 min. treatment; Lane 1-3, 55 sec. treatment]. The spray application treatment time is preferably 55 seconds or longer, commercially preferably 1 minute or longer. At this time, commercial suitability was determined based on the use of hydrogen peroxide as a second agent and the length of the perm hair after washing once was about 22 cm.

### 6) Use of 90°C Electrolyzed Water as Second Agent of Perm

Tap water was used as the raw material water used to compare the wave forming power of electrolyzed water generated by electrolysis at 90°C. 400 ml of 90°C tap water was put into an electrolyzed water generator with an output of 27W (9V, 3A) (Ecowell, Korea) and electrolyzed water generation process was performed for 20 minutes to obtain electrolyzed water, which was used in the experiment. The perm process was performed at normal room temperature (cold perm). In perm treatment, commercially available Hair 119 (Eson Chemical, Korea) was used as the first agent, and the main ingredients are cysteine HCL and cysteamine HCL. Electrolyzed hydrogen water was used as the second agent.

About 50 strands of natural black hair were fixed and cut to 27 cm in length and used in the experiment. The perm process was performed as follows. The natural black hair was fixed and washed according to the above standards. After washing, the dried hair bundle was reduced by treatment with the first agent (Hair 119 Clinic SuBoon Perm, Eson Chemical, Korea) at 37°C for 25 minutes. After the reduction process, the first agent was removed by washing with distilled water. The washed hair was wound and fixed on a rod with a diameter of 8 mm. Electrolyzed water was put into a sprayer and applied while continuous spraying to the hair fixed on the rod at a spray speed of 10 ml/min to reoxidize (Repeated application experiments were performed with a pipette of 5 ml per time). After the application was finished, the rod was removed. In order to measure the perm efficiency, the length was measured, and photos were taken after washing (FIG. 3E).

The numbers shown in FIG. 3E are results of a change in length of hair (natural black hair) during washing of the perm hair according to the second electrolyzed water treatment during perm treatment [Lane 1: 20 sec. treatment; Lane 2: 25 sec. treatment; Lane 3: 30 sec. treatment; Lane 4: 35 sec. treatment; Lane 5: 40 sec. treatment; Lane 6: 45 sec. treatment] . The spray-type continuous application treatment time of electrolyzed water generated by electrolysis at 90°C requires 40 seconds or longer, commercially desirable 45 seconds or longer.

### 3. Perm Efficiency according to Manufacturing Apparatus

### 1) Comparison of Perm Efficiency of Electrolyzed Hydrogen Water according to Apparatus Performance

In order to find out whether the difference in apparatus output affects the formation of the perm, the raw material water was adjusted to 20°C and perm experiment was performed. The raw material water used for generating hydrogen water was prepared as follows. The temperature of 20°C of 400 ml of NaCl 0.05% (w/v) solution was maintained. 400 ml of the raw material water was put into a hydrogen water generator (Grentech, Korea) with an output of 24W (12V, 2A) and hydrogen generation process was performed for 20 minutes to obtain A hydrogen water. The raw material water was put into a hydrogen water generator (Solco, Korea) with an output of 10W (5V, 2A) and hydrogen generation process was performed for 20 minutes to obtain B hydrogen water, which were used as hydrogen water.

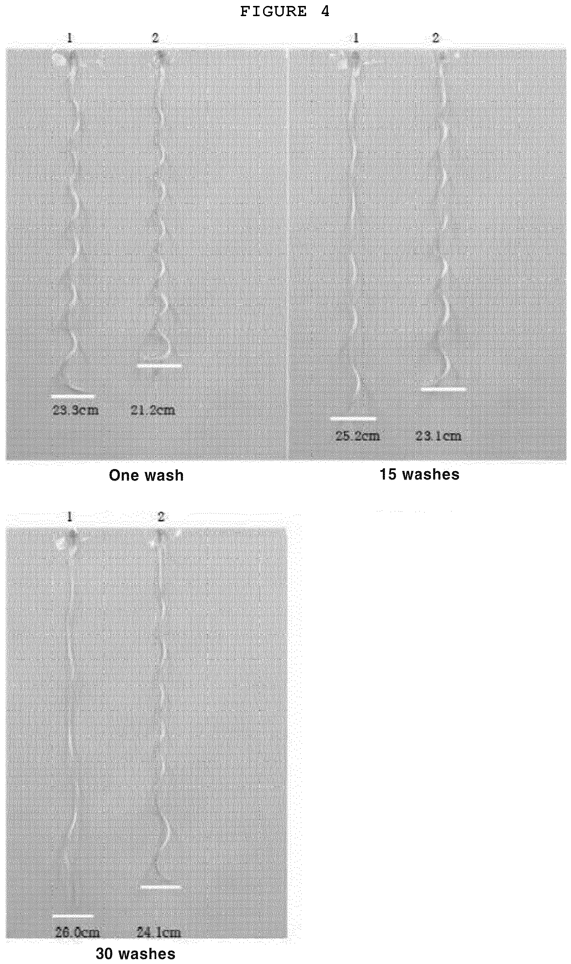

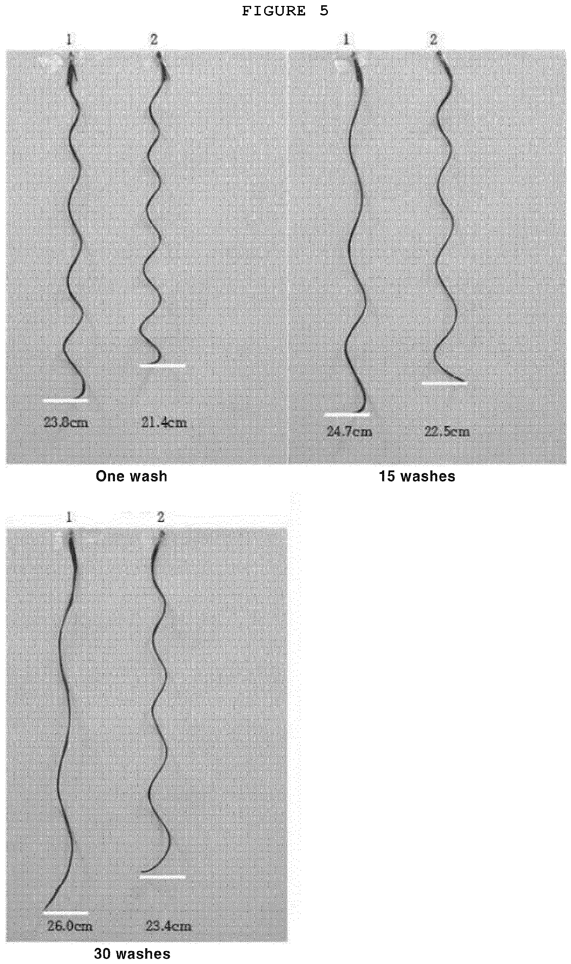

About 50 strands of natural black hair and blonde hair were fixed and cut to 27 cm in length and used in the experiment. The perm process was performed as follows. After washing, the dried hair bundle was reduced by treatment with the first agent (Hair 119 Clinic SuBoon Perm, Eson Chemical, Korea) at 37°C for 25 minutes. After the reduction process, the first agent was removed by washing with distilled water. The washed hair was wound and fixed on a rod with a diameter of 8 mm and reoxidized by applying each hydrogen water 10 times at one-minute intervals. When repeatedly applied, it was treated with a pipette at a rate of 5 ml per time. After the application was finished, the rod was removed. In order to measure the perm efficiency, the length was measured, and photos were taken. To check the durability, after washing with shampoo (Natural Shampoo, Repit, Korea) was carried out 15 times and 30 times, the length was measured, and photos were taken (FIGs. 4 and 5).

As shown in FIG. 4, in the case of blonde hair, the wave efficiency of hydrogen water prepared by A hydrogen water generator with an output 24W (12A, 2A) (Lane 2 in FIG. 4) was better than the hydrogen water prepared by B hydrogen water generator with an output of 10W (5V, 2A) (Lane 1 of FIG. 4) in washing once, 15 times, and 30 times and the wave efficiency was good even after washing 30 times. When washing hair once every two days after perm treatment, the wave is maintained well for at least 2 months, so commercialization is sufficient. On the other hand, in the case of B hydrogen water generator, the wave is not maintained well even after 15 washings, so it is judged not to be non-commercia.

As shown in FIG. 5, in the case of natural black hair, the wave efficiency of hydrogen water prepared by A hydrogen water generator with an output 24W (12A, 2A) (Lane 2 in FIG. 5) was better than the hydrogen water prepared by B hydrogen water generator with an output of 10W (5V, 2A) (Lane 1 of FIG. 5) in washing once, 15 times, and 30 times and the wave efficiency was good even after washing 30 times. When washing hair once every two days after perm treatment, the wave is maintained well for at least 2 months, so commercialization is sufficient. Conversely, in the case of hydrogen water of B hydrogen water generator, the perm wave is relatively poor even after 15 washes, so it can be seen that the commerciality is lowered (FIG. 5).

### 2) Comparison of Perm Efficiency of Electrolyzed Hydrogen Water and Electrolyzed Water with Similar Output

In order to find out if the electrolyzed water and hydrogen water generated by apparatus with similar output affects the formation of the perm, the raw material water was adjusted to 20°C and perm experiment was performed. The raw material water used for generating hydrogen water was prepared as follows. The temperature of 20°C of 400 ml of NaCl 0.05% (w/v) solution was maintained. 400 ml of the raw material water was put into a hydrogen water generator (Grentech, Korea) with an output of 24W (12V, 2A) and hydrogen generation process was performed for 20 minutes to obtain A hydrogen water. The raw material water was put into an electrolyzed water generator (Ecowell, Korea) with an output of 27W (9V, 3A) and electrolyzed water generation process was performed for 20 minutes to obtain C electrolyzed water, which was used as electrolyzed water.

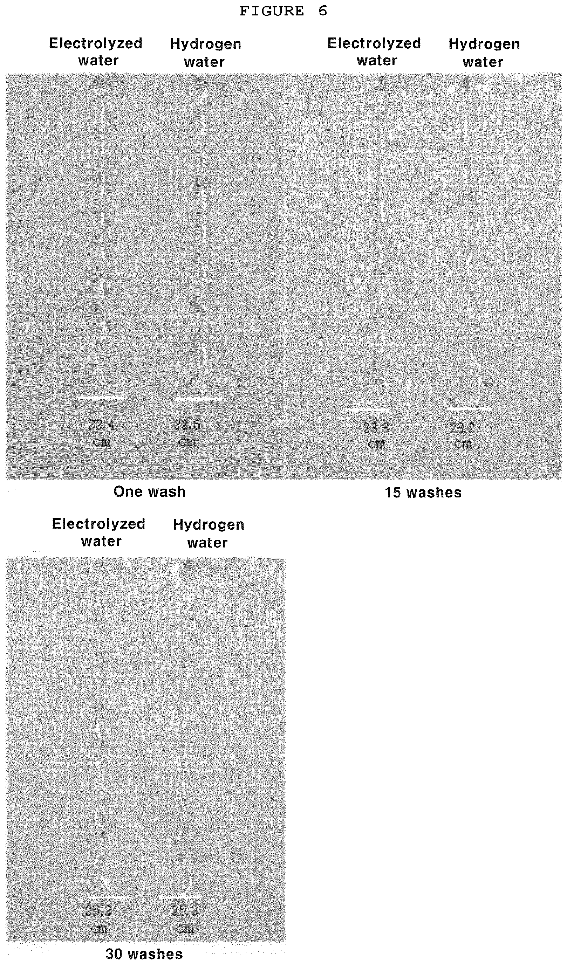

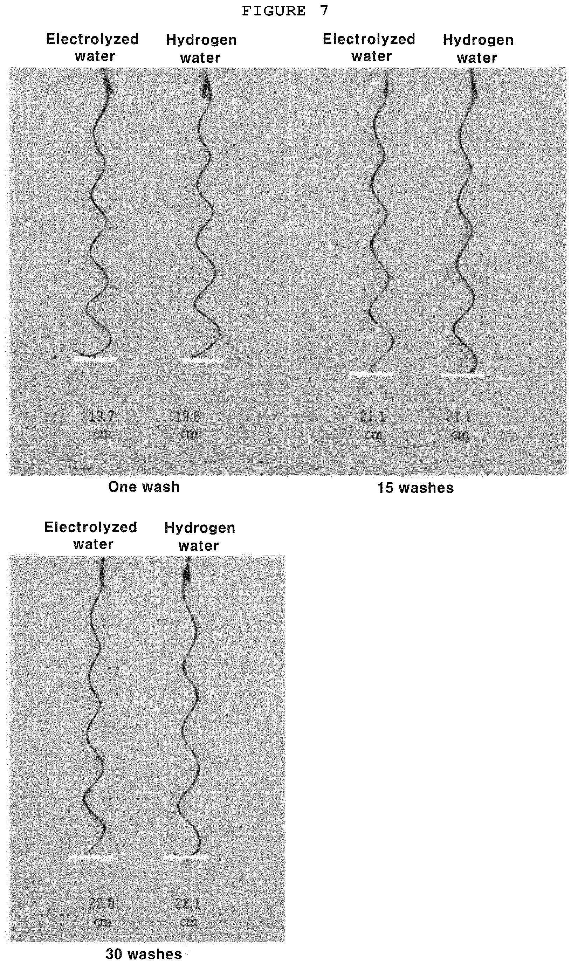

About 50 strands of natural black hair and blonde hair (Moresso, UK) were fixed and cut to 27 cm in length and used in the experiment. The perm process was performed as follows. After washing, the dried hair bundle was reduced by treatment with the first agent (Hair 119 Clinic SuBoon Perm, Eson Chemical, Korea) at 37°C for 25 minutes. After the reduction process, the first agent was removed by washing with distilled water. The washed hair was wound and fixed on a rod with a diameter of 8 mm and reoxidized by applying each hydrogen water 10 times at one-minute intervals. When repeatedly applied, it was treated with a pipette at a rate of 5 ml per time. After the application was finished, the rod was removed. In order to measure the perm efficiency, the length was measured, and photos were taken. To check the durability, after washing with shampoo (Natural Shampoo, Repit, Korea) was carried out 15 times and 30 times, the length was measured, and photos were taken (FIGs. 6 and 7).

As shown in FIG. 6, in the case of blonde hair, the wave efficiency of hydrogen water prepared by A hydrogen water generator with an output 24W (12A, 2A) and electrolyzed water prepared by C electrolyzed water generator with an output of 27W (9V, 3A) is similarly good in one washing, 15 washes, and 30 washes. When washing hair once every two days, the wave is maintained well for at least 2 months, so commercialization was found to be sufficient (FIG. 6).

As shown in FIG. 7, in the case of natural black hair, like blonde hair, both hydrogen water and electrolyzed water have good wave efficiency even when washing 30 times, and thus, when washing hair once every two days, the wave is maintained well for 2 months, so commercialization is sufficient (FIG. 7). As such, hydrogen water and electrolyzed water generated by apparatus with similar outputs showed similar perm wave efficiency.

### 4. Perm Efficiency according to pH of Perm Second Agent

### 1) Perm Efficiency of Electrolyzed Hydrogen Water and Electrolyzed Water according to pH

In order to find out whether a change in pH affects the perm formation, a perm experiment was conducted by dividing the raw material water into the ranges of pH 4-10, pH 1-4, pH 07, and pH 10-12.

The water used for generating hydrogen water and electrolyzed water was prepared as follows. NaCl was dissolved in 400 ml of distilled water so that the concentration of the electrolyte was 0.05% (w/v). 400 ml of the solution was put into a hydrogen water generator (Grentech, Korea) with an output of 24W (12V, 2A) and hydrogen generation process was performed for 20 minutes to obtain hydrogen water, which was used as hydrogen water. The solution was put into an electrolyzed water generator (Ecowell, Korea) with an output of 27W (9V, 3A) and electrolyzed water generation process was performed for 20 minutes to obtain electrolyzed water. Then, in the range of pH 4-10, 0.05% (w/v) NaCl solution adjusted to each pH was experimented with hydrogen water and electrolyzed water prepared by a hydrogen water generator and an electrolyzed water generator. In addition, at pH 0.7, 1, 2, 3, 4, 10, 11, and 12 and pH 11.25, 11.50, 11.75, and 12.25, hydrogen water and electrolyzed water were prepared, 10M NaOH and 10M HCl were added to make each pH.

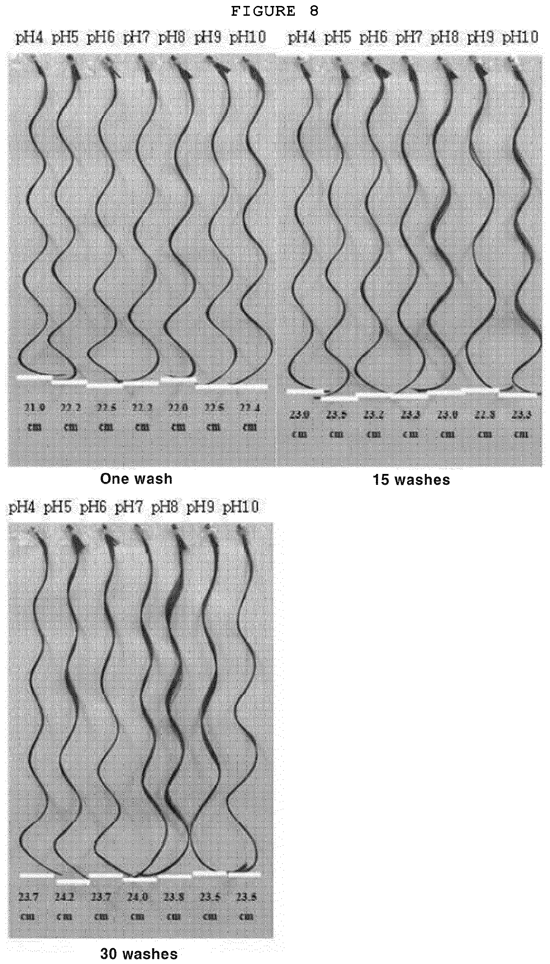

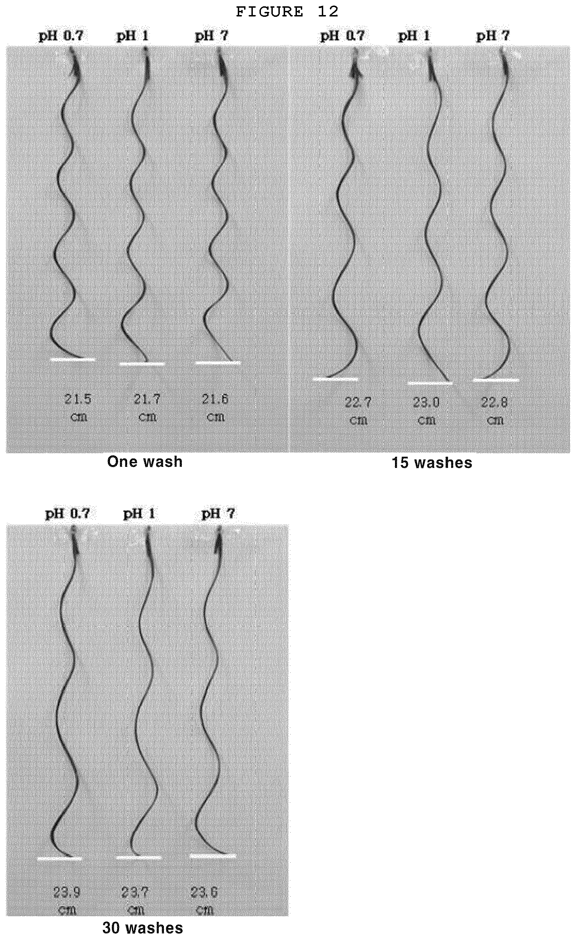

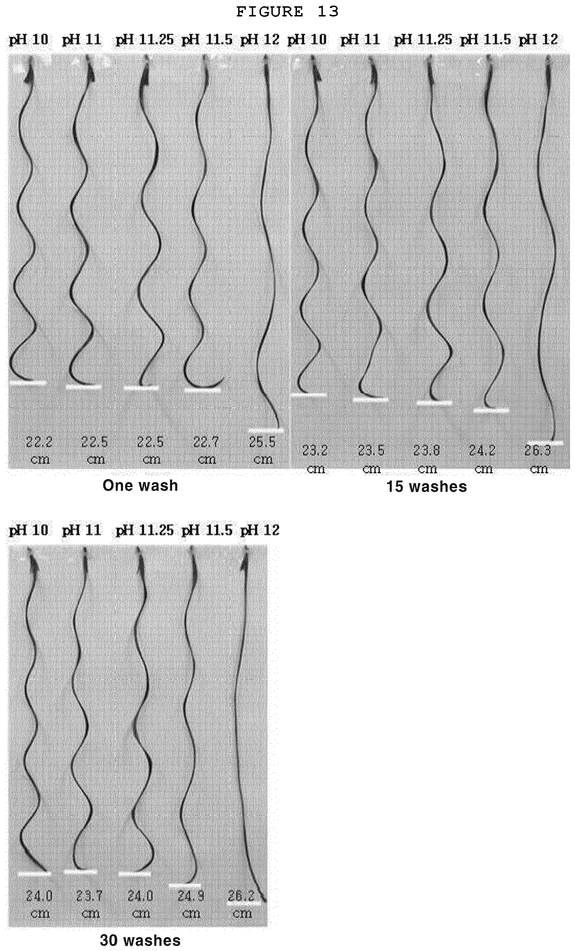

About 50 strands of natural black hair was fixed and cut to 27 cm in length and used in the experiment. The perm process was performed as follows. After washing, the dried hair bundle was reduced by treatment with the first agent (Hair 119 Clinic SuBoon Perm, Eson Chemical, Korea) at 37°C for 25 minutes. After the reduction process, the first agent was removed by washing with distilled water. The washed hair was wound and fixed on a rod with a diameter of 8 mm and reoxidized by applying each hydrogen water 10 times at one-minute intervals. After the application was finished, the rod was removed. In order to measure the perm efficiency, the length was measured, and photos were taken. To check the durability, after washing with shampoo (Natural Shampoo, Repit, Korea) was carried out 15 times and 30 times, the length was measured, and photos were taken (FIGs. 8 to 13).

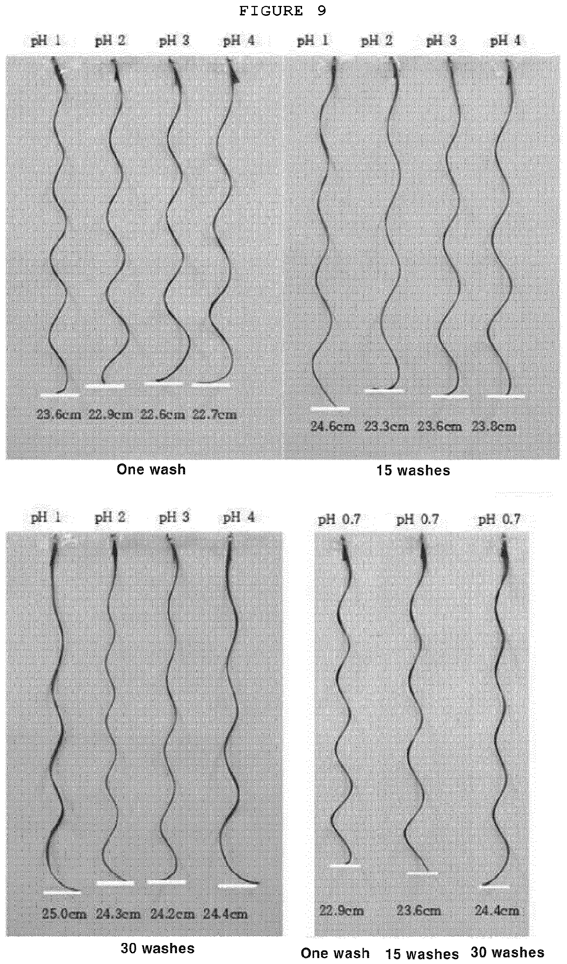

As shown in FIG. 8, there was little difference in wave efficiency in one wash, 15 washes, and 30 washes of perm hair according to pH 4-10 of hydrogen water. In addition, as shown in FIG. 9, there was little difference in one wash, 15 washes, and 30 washes of pH 1-4 of hydrogen water. In the case of pH 0.7, it slightly increased according to the number of 15 washes and 30 washes, and it is the same as the value of pH 1-4.

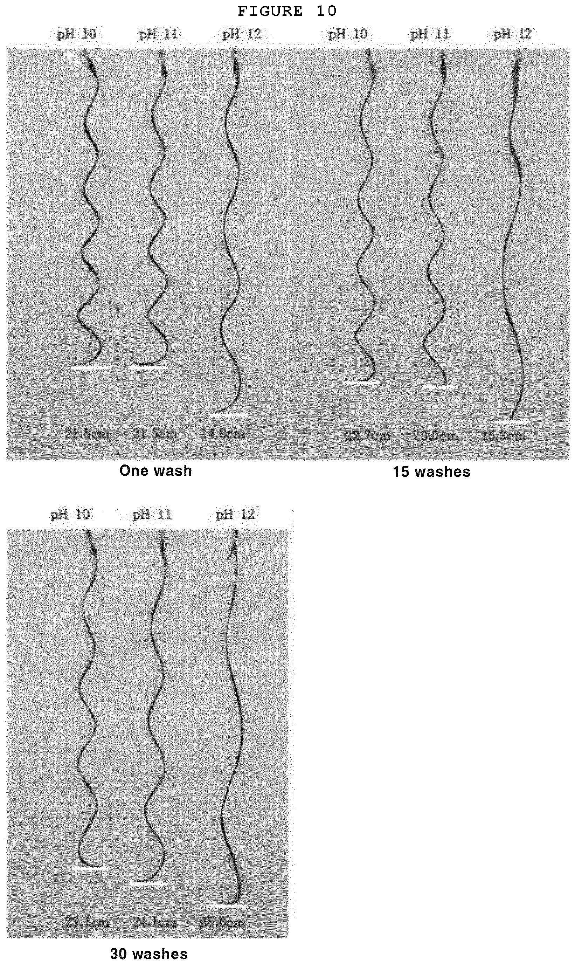

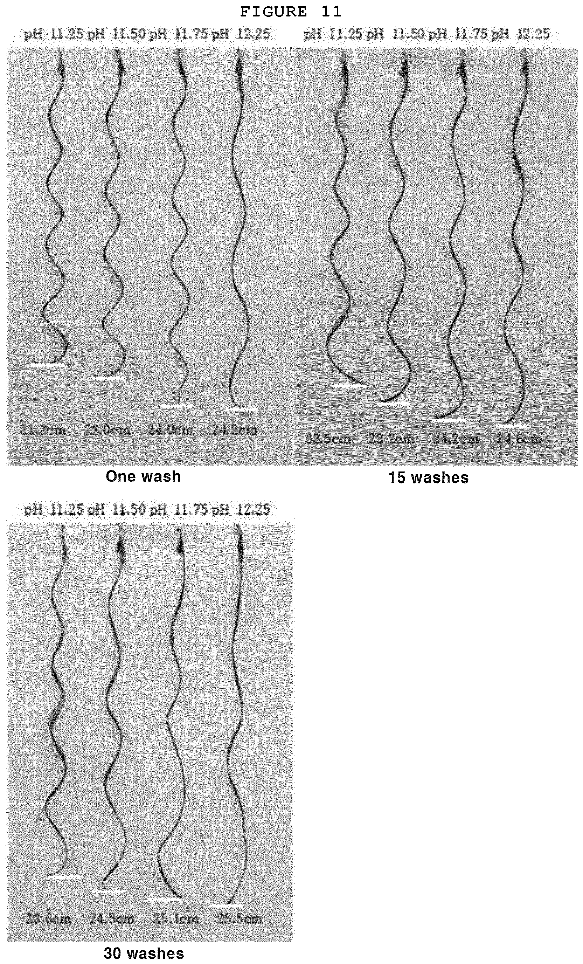

As shown in FIG. 10, at pH 10-12 of hydrogen water, pH 10 and 11 showed almost the same wave efficiency until one wash and 15 washes, and at 30 washes, wave efficiency tends to be slightly reduced. However, at pH 12, there was a lot of difference from pH 11. Here, the pH was subdivided into pH 11.25, 11.50, 11.75, and 12.25, and as a result of the re-experiment (FIG. 11), the wave efficiency at one wash, 15 washes, and 30 washes of perm hair appeared to some extent up to pH 11.50. It was found that preferably, the wave efficiency appears well up to pH 11.25. Therefore, the hydrogen water to pH 1-11.50 has good wave efficiency, and preferably to pH 1-11.25.

### 5. Perm Efficiency according to Temperature of Perm Second Agent

### 1) Perm Efficiency of Electrolyzed Hydrogen Water and Electrolyzed Water according to Temperature

In order to find out whether temperature of raw material water affects the perm formation, a perm experiment was conducted with the raw material water adjusted to as 20-29°C and 5-20°C, respectively. The water used for generating hydrogen water and electrolyzed water was prepared as follows. 400 ml of NaCl 0.05% (w/v) solution was heated with Waterbath (ChangSin, Korea) so that temperature was 20, 40, 60, 80, and 90°C. In addition, 400 ml of NaCl 0.05% (w/v) solution was cooled so that temperature was 5, 10, 15, and 20°C. 400 ml of each of them was put into a hydrogen water generator (Grentech, Korea) with an output of 24W (12V, 2A) and hydrogen generation process was performed for 20 minutes to obtain hydrogen water. In addition, the solution was put into an electrolyzed water generator (Ecowell, Korea) with an output of 27W (9V, 3A) and electrolyzed water generation process was performed for 20 minutes to obtain electrolyzed water, which was used as electrolyzed water. In order to prevent temperature change during the generation process, a plastic container with ice was placed inside the apparatus to control the temperature.

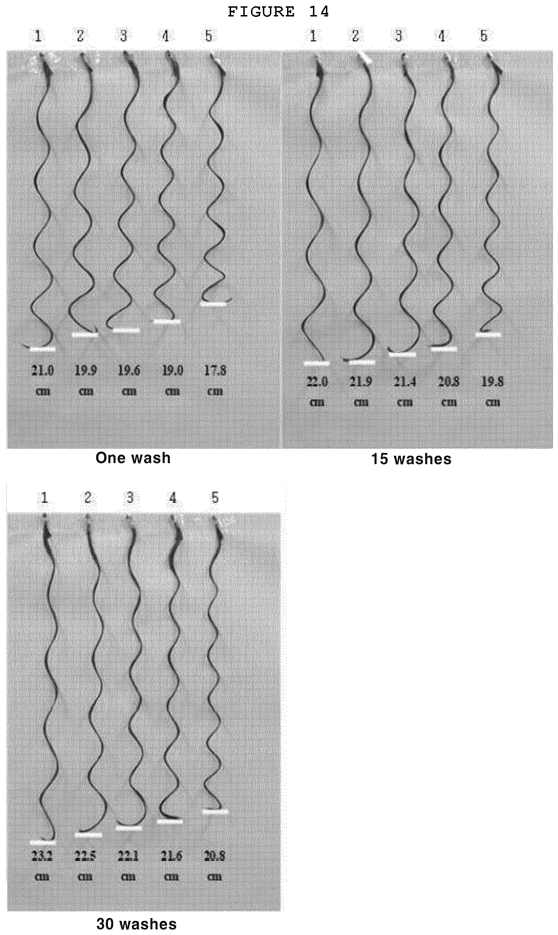

About 50 strands of natural black hair was fixed and cut to 27 cm in length and used in the experiment. The perm process was performed as follows. After washing, the dried hair bundle was reduced by treatment with the first agent (Hair 119 Clinic SuBoon Perm, Eson Chemical, Korea) at 37°C for 25 minutes. After the reduction process, the first agent was removed by washing with distilled water. The washed hair was wound and fixed on a rod with a diameter of 8 mm and reoxidized by applying each hydrogen water 10 times at one-minute intervals. After the application was finished, the rod was removed. In order to measure the perm efficiency, the length was measured, and photos were taken. To check the durability, after washing with shampoo (Natural Shampoo, Repit, Korea) was carried out 15 times and 30 times, the length was measured, and photos were taken (FIGs. 14 to 16).

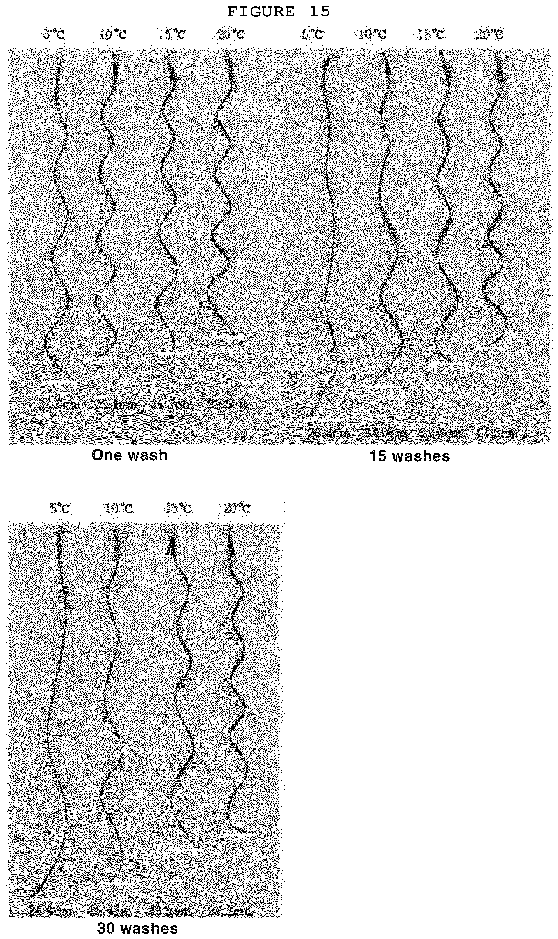

As shown in FIG. 14, when using hydrogen water up to 20-29°C, the wave efficiency improved in proportion to the temperature in one wash, 15 washes, and 30 washes (Lane 1: 20°C; lane 2: 40°C; lane 3: 60°C; lane 4: 80°C; lane 5: 90°C). As shown in FIG. 15, when the temperature was subdivided into 5°C, 10°C, 15°C, and 20°C and hydrogen water was used, the wave efficiency was good at 15°C in one wash, 15 washes, and 30 washes, but showed a tendency to drop slightly at 10°C. Therefore, it can be seen that the temperature of hydrogen water can be used up to 10-90°C, but preferably up to 15-19°C has good wave efficiency (FIG. 15).

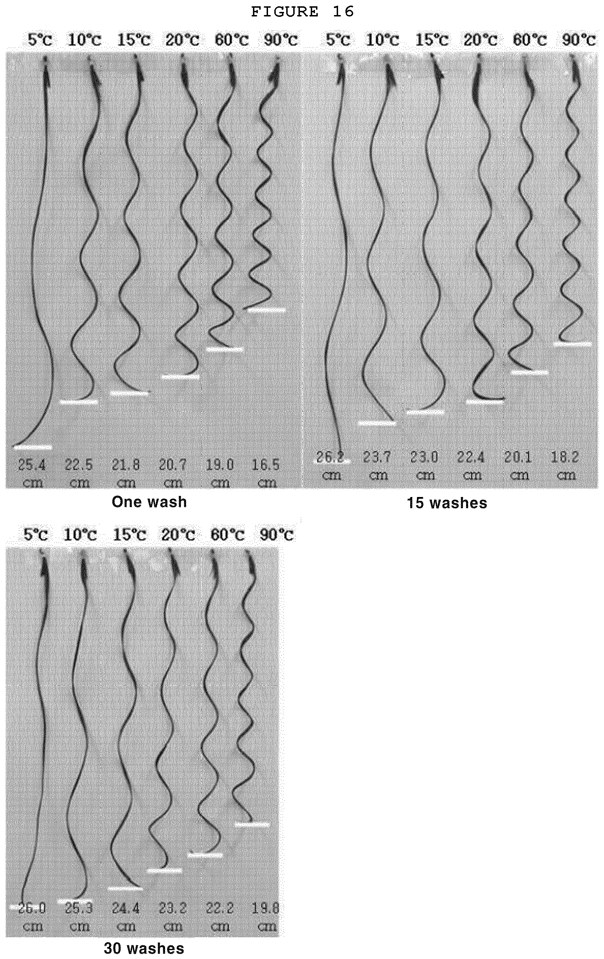

As shown in FIG. 16, when the temperature was subdivided into 5°C, 10°C, 15°C, 20°C, 60°C, and 90°C and electrolyzed water was used, the wave efficiency was good at 15°C in one wash, 15 washes, and 30 washes, but showed a tendency to drop slightly at 10°C. Therefore, it can be seen that the temperature of electrolyzed water can be used up to 10-90°C, but preferably up to 15-19°C has good wave efficiency (FIG. 16). In summary, the higher the temperature, the better the wave efficiency, but if it is above 40°C, a user should be careful when it comes into contact with the scalp.

### 6. Effect of Straight Perm of Curly Hair in Electrolyzed Hydrogen Water and Electrolyzed Water

The first agent used in straight perm was Standard of Hair Chalang Chalang Two Way Straight (UCL, Incheon). The second agent used in the perm was a mixture of Standard of Hair Chalang Chalang Two Way Straight (UCL, Incheon) and xanthan gum in each hydrogen water and electrolyzed water.

Tap water was used to generate the used electrolyzed hydrogen water and electrolyzed water. 400 ml of tap water was put into a hydrogen water generator with an output of 24W (12V, 2A) (Grentech, Korea) and hydrogen water generation process was performed for 20 minutes to obtain hydrogen water, which was used as hydrogen water. Tap water was put into an electrolyzed water generator with an output of 27W (9V, 3A) (Ecowell, Korea) and electrolyzed water generation process was performed for 20 minutes to obtain electrolyzed water, which was used as electrolyzed water. To each of these, xanthan gum was added to 0.5% and mixed to form a gel.

The perm process proceeded as follows. The first agent was applied to the hair and softened at 37°C for 30 minutes. After softening, the hair was washed to remove the first agent, each of the xanthan gum mixture of the second agent or electrolyzed water was applied and oxidized for 10 minutes. After the oxidation process was completed, the hair was washed, photographed, and measured.

### 1) Straight Perm Treatment Using Electrolyzed Hydrogen Water

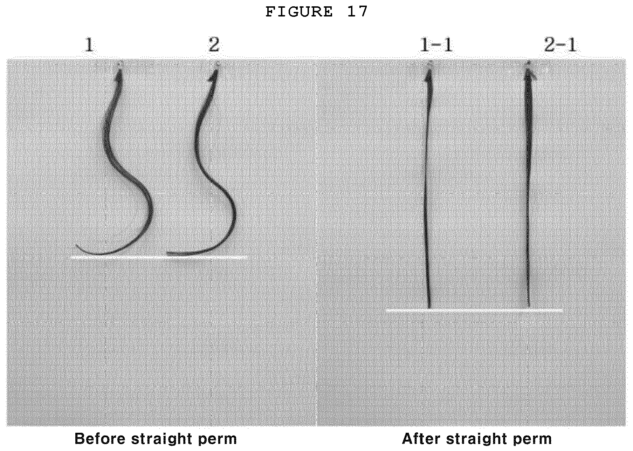

The hair was made by fixing about 50 strands of curly hair, and two bundles with the most similar curvature were selected and used in the experiment. In FIG. 17, lanes 1 and 2 are hair bundles before straight perm treatment, and lanes 1-1 and 2-1 are hair bundles after straight perm treatment. As shown in FIG. 17, it can be seen that curly hair became straight hair after straight perm treatment (Lane 2-1), there was no difference in the effect from the case of using hydrogen peroxide as the second agent (lane 1-1) (FIG. 17).

### 2) Effect of Straight Perm Treatment of Electrolyzed Water

In a straight perm using electrolyzed water for curly hair, lanes 3 and 4 are pre-experimental hair bundles and lanes 3-1 and 4-1 are hair bundles after perm treatment. In both lane 3-1 treated with hydrogen peroxide as the second agent and lane 4-1 treated with electrolyzed water containing xanthan gum, curly hair was well straightened.

### 7. Comparison of Cysteic Acid Generation by FTIR Analysis of Perm Hair

In the perm treatment, when the cystine bond (disulfide bond) of the hair is oxidized to cysteic acid, it cannot be reformed the cystine bond with the damaged hair. To determine the degree of damage to the perm hair, the cysteic acid ratio can be measured through ATR-FTIR.

ATR-FTIR was used to measure how much cysteic acid was prepared compared to hydrogen peroxide when hydrogen water and electrolyzed water were treated as the second perm agent. About 50 strands of natural hair was fixed and cut to 27 cm in length and used in the experiment. The perm process was performed as follows. After washing, the dried hair bundle was reduced by treatment with the first agent (Hair 119 Clinic SuBoon Perm, Eson Chemical, Korea) at 37°C for 25 minutes. After the reduction process, the first agent was removed by washing with distilled water. The washed hair was wound and fixed on a rod with a diameter of 8 mm and reoxidized by applying hydrogen water and electrolyzed water as each second agent 10 times at one-minute intervals. Hydrogen peroxide (Hair 119 Clinic SuBoon Perm, Eson Chemical, Korea) was treated once for 5 minutes with a general perm treatment method. After the application was finished, the rod was removed. It was washed once (Natural Shampoo, Repit, Korea) and used as a hair sample.

Here, a 0.05% NaCl solution prepared by adding NaCl to distilled water was used to prepare electrolyzed hydrogen water and electrolyzed water used in perm treatment. 400 ml of the solution was put into an electrolyzed water generator with an output of 24W (12V, 2A) (Grentech, Korea) and hydrogen water generation process was performed for 20 minutes to obtain a hydrogen electrolyzed water. The solution was put into an electrolyzed water generator with an output of 27W (9V, 3A) and electrolyzed water generation process was performed for 20 minutes to obtain electrolyzed water, which was used as electrolyzed water.

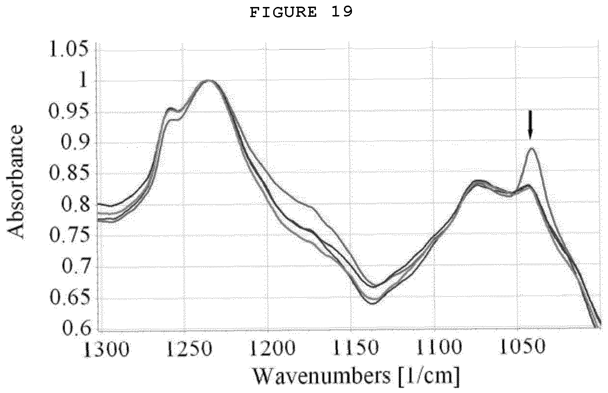

The hair sample was measured using ATR-FTIR spectrophotometer (FT/IR-4100, Jasco, Japan). The wavenumber was adjusted to 4,000 to 600cm⁻¹ and measured at intervals of 1cm⁻¹. The average spectrum was obtained by measuring two experimental samples each. Spectragryph 1.2(Dr. Friedrich Menges Softwareentwicklung, Germany) was used for FTIR spectral irradiation and data conversion. The ratio of cysteine acid in hair was measured by normalizing based on the amide III peak height of 1242cm⁻¹ of an average spectrum, and then comparing the peak height of 1040cm⁻¹. The amount of cysteic acid was compared through infrared rays of 1040cm⁻¹ absorbed by S-O of cysteic acid through bending vibration. ATR-FTIR spectrum is shown in Table 2 below and FIG. 19. Table 2 is a numerical result of the measured peak height of 1040cm⁻¹.

**Table 2**

| Run | Control group | Hydrogen peroxide | Hydrogen water | Electrolyzed water |
|---|---|---|---|---|
| Peak height | 0.8226 | 0.8867 | 0.8261 | 0.8273 |

As a result of the measurement of the amount of cysteic acid prepared by the perm treatment, hydrogen water increased by 0.0035 and electrolyzed water increased by 0.0047 whereas hydrogen peroxide increased by 0.0641. In other words, compared to the increase in hydrogen peroxide, it was found that the hydrogen water perm prevented 94.5% of damage, and the electrolyzed water prevented 92.7% of damage. The control group is hair before perm treatment.

### 8. Evaluation of Feasibility of Commercial Perm Hair by Perm Treatment of Human Hair Wig - Perm Treatment of Human Hair Wig using Electrolyzed Water

In order to verify the effectiveness of the perm treatment using electrolyzed water, electrolyzed water perm treatment was performed on a wig with 100% human hair, assuming that it was performed on a real person at a beauty salon.

The perm agent used in the experiment was generally used in hair salons and selected in consideration of stability and excellence, and C company (Cosmocos, Korea) product of which reducing component is thioglycolate was used. Tap water of 27°C was put into an electrolyzed water generator with an output of 27W (9V, 3A) (Ecowell, Korea) and electrolyzed water generation process was performed for 20 minutes to obtain electrolyzed water, which was used as the second agent. After applying a first agent to a 100% human hair wig, croquignole-type winding on a round rod with a diameter of about 10mm in a horizontal section, sealed with a plastic cap, in an incubator at 37°C for 15 minutes, and left at room temperature for 15 minutes, without intermediate washing, 200 ml of oxidation agent (electrolyzed water) was put into a spray and sprayed (takes about 5 minutes) and then oxidized for 10 minutes. After that, 200 ml was again put into the sprayer, sprayed, applied, and oxidized for 10 minutes, that is, applying twice 400 ml in total. Then, the rod was removed, rinsed in running water, and then dried naturally in the direction of gravity.

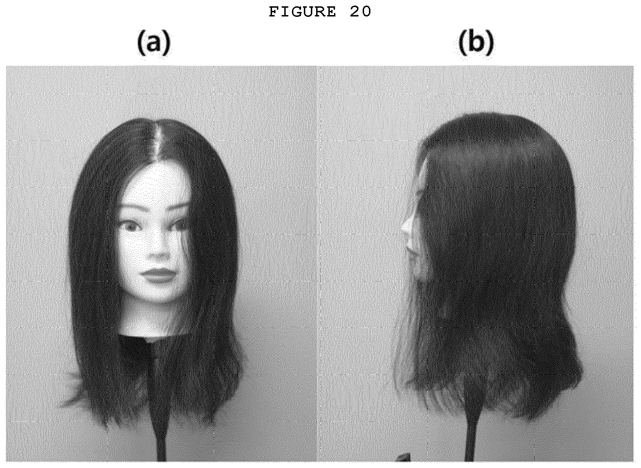

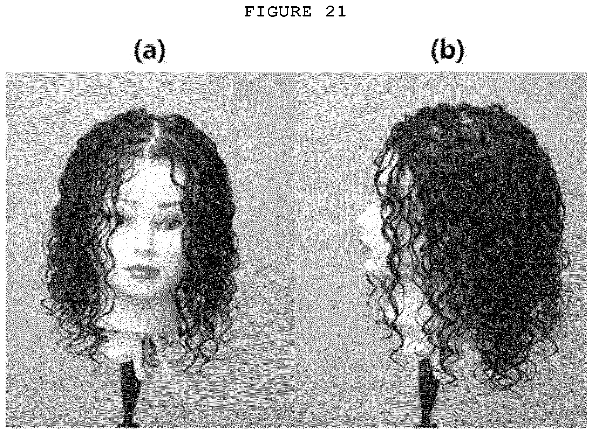

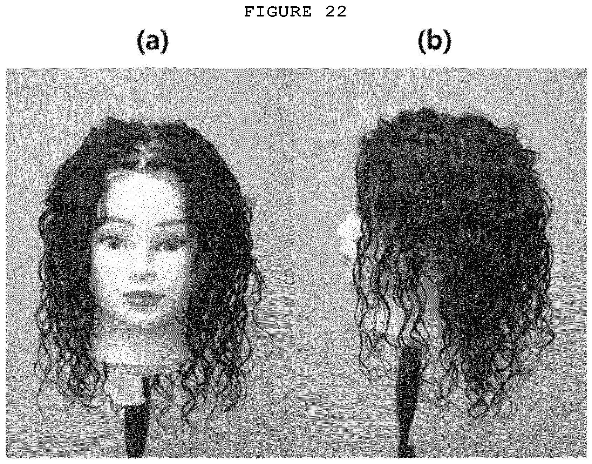

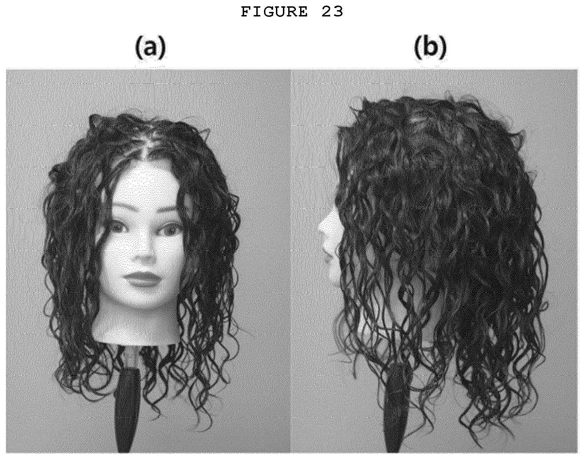

To see the durability of the perm, the wig was photographed before perm, after one wash, after 15 washes, and 30 washes (FIGs. 20 to 23) . Shampoo (Haedeun Cosmetics, Korea) was used for washing, and the shampoo was diluted in tap water, applied to the wig, rinsed with running tap water, and then dried naturally in the direction of gravity. If washing once every two days in real life, it can be seen that the wave is maintained well for 3 months. Therefore, it can be seen that the method of using hydrogen water and electrolyzed water as a perm second agent in various experiments and human wig perm experiments is a method with sufficient commerciality that can be applied immediately to the field.

### 9. Antifungal Effect of Electrolyzed Water against Dandruff Bacterium Malassezia furfur

*Malassezia furfur* in the scalp causes the stench and dandruff of the scalp. *M. furfur* is a lipophilic yeast that generates lipase in the skin with many sebaceous glands and decomposes sebum to generate free fatty acid. This action damages the skin barrier of the scalp and causes various skin disease. Scalp diseases caused by *M. furfur* include atopic dermatitis, seborrheic dermatitis, pityriasis versicolor, folliculitis, dandruff, and fungemia, etc. As a treatment for diseases caused by *M. furfur,* there is a treatment using selenium sulfide, but there are adverse effects such as vomiting, abdominal pain, lethargy, skin irritation, hair bleaching, and hair loss, etc.

*M. furfur* may be contaminated when hair is permed or cut in a hairdresser or barber shop, in order to confirm the dandruff relief effect of electrolyzed water and hydrogen water, antifungal activity against *M. furfur,* the cause of dandruff, was tested. For the experiment, *M. furfur* KCTC 7545 was purchased from KCTC and used, and modified Sabouraud's dextrose agar was used for culturing *M. furfur.* Modified Sabouraud's dextrose agar (olive oil 1%, Tween 80 1%, dextrose 4%, peptone 1%, agar 2%) was mixed with distilled water to be 1L, autoclaved at 121°C for 15 minutes, cooled to 50°C, and mixed and dispensed to prevent the layer from separating.

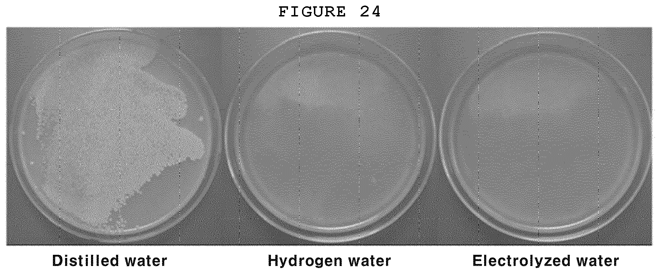

As a fungal fluid for inoculating the train in the corresponding medium, 0.25 cm² of a culture medium in which *M*. *furfur* was sufficiently cultured was diluted into 10 ml of distilled water, electrolyzed water, and hydrogen water, respectively. At this time, distilled water, electrolyzed water, and hydrogen water were shaken for 5 minutes to ensure sufficient sterilization, and these were used as a bacterial smear solution. The prepared fungal fluid was injected into the medium by 0.2 ml each, smeared, and then cultured at 27°C for 3 days and observed. As a result, distilled water Petri dish was filled of small *M*. *furfur* colonies like millet. However, *M. furfur* colonies did not appear in the hydrogen water or electrolyzed water petri dish (See FIG. 24). Therefore, it can be seen that the electrolyzed water or hydrogen water have antibacterial properties against *M*. *furfur.*

### 10. Effect of Inhibiting Dye Elution by Replacing Perm Second Agent with Electrolyzed Water in Perm Treatment Process of Dyed Hair

The process of perm treatment is treated with a reduction agent, which is a first agent of perm, and then an oxidation agent (hydrogen peroxide), which is the second agent of perm, is used to fix it in the desired shape. However, in general, when dyed hair is permed, the dye of the hair is eluted in during this process, and the color of the dyed hair is mostly faded compared to before the perm treatment. Therefore, in the perm treatment process of dyed hair, by replacing hydrogen peroxide, which is the second agent of perm, with electrolyzed water, the hair dye can be prevented from being eluted compared to the existing perm treatment method for dyed hair, thereby improving color fading of perm treated dyed hair.

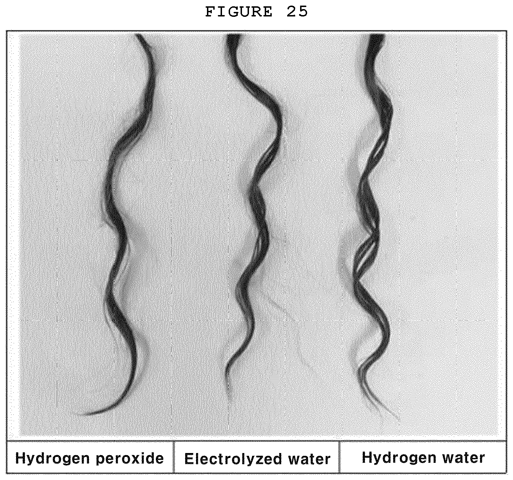

The hair used for dark brown dyeing is washed once to remove foreign substances and contaminants, dried, treated with a bleaching agent (multi blonde lightening powder (Wella, USA) and Welloxon perfect cream developer (Wella, USA)) mixed in a 1:2 ratio is used) for 15 minutes, then washed, and used. After washing, a dark brown dye was applied to the entire hair left at 37°C for 15 minutes and washed with distilled water. Estetica Multifunction (Somang Cosmetics, Korea) was used as a perm agent, the main component of the first agent is cysteine, and the main component of the second agent is hydrogen peroxide. First, a first agent was applied to the dyed hair and treated for 15 minutes to proceed with the softening process. After that, the hair was fixed on a rod with a diameter of 8 mm, and 10 ml of perm second agent (hydrogen peroxide), hydrogen water and electrolyzed water were applied to each of the three samples and left for 7 minutes. The hair that went through the perm process was washed once with tap water, dried, and went through a colorimetric process (Table 3 and FIG. 25).

In all three samples, the hair dye was slightly eluted when the perm first agent was applied in the perm treatment. In addition, the dye did not elute when applied with electrolyzed water and hydrogen water as the second agent, but when hydrogen peroxide was applied, the dye also eluted, and discoloration appeared. In addition, when washing with tap water once, dye was eluted in the hydrogen peroxide group, and electrolyzed water and hydrogen water had almost no elution of the dye.

The color of the dyed hair was measured using a colorimeter spectrophotometer CM-2500d (Konica Minolta Sensing, Inc., Japan). FIG. 25 shows the color of the dyed hair before the perm treatment and the hair after the perm treatment and washing once. There was little difference in the hair before the perm treatment, and there was a difference in the thickness (L) after the perm treatment. In the case of washing once after the perm treatment, 2.01% of the dye was eluted in the electrolyzed water group, and 4.74% was eluted in the hydrogen water group. However, 11.43% was eluted in hydrogen peroxide group. Therefore, it can be seen that, unlike hydrogen peroxide, a dye elution is very suppressed when dyed hair is perm treated with electrolyzed water or hydrogen water.

In addition, in the three bleached hairs, the hydrogen peroxide group had split ends, and the overall hair surface was rough. On the other hand, the electrolyzed water group and the hydrogen water group did not have split ends and the hair looked relatively neat and smooth, but the electrolyzed water group was better.

**Table 3**

| **Hair before perm** | **Hydrogen peroxide** | **Electrolyzed water** | **Hydrogen water** |
|---|---|---|---|
| L | 27.56 | 27.29 | 27.10 |
| a | 51.05 | 51.46 | 50.43 |
| b | 16.28 | 16.37 | 17.01 |

| **Hair after perm** | **Hydrogen peroxide** | **Electrolyzed water** | **Hydrogen water** |
|---|---|---|---|
| L | 31.12 | 27.85 | 28.44 |
| a | 52.04 | 51.51 | 51.03 |
| b | 18.27 | 17.08 | 17.41 |

### 11. Comparison of Effect of Using Electrolyzed Water for Perm Treatment of Dyed Hair as Second Agent and for Washing

### 1) Wave Efficiency of Dyed Hair

Tap water was used as raw material water used to compare the wave formation ability. 400 ml of tap water of 25°C was put into a hydrogen water generator with an output of 24W (12V, 2A) (Grentehch, Korea) and hydrogen water generation process was performed for 20 minutes to obtain hydrogen water, which was used in the experiment. The dyeing process was performed at normal room temperature. The commercially available Shining Essence 7C (Miseenscene, Korea) was used as a dye.

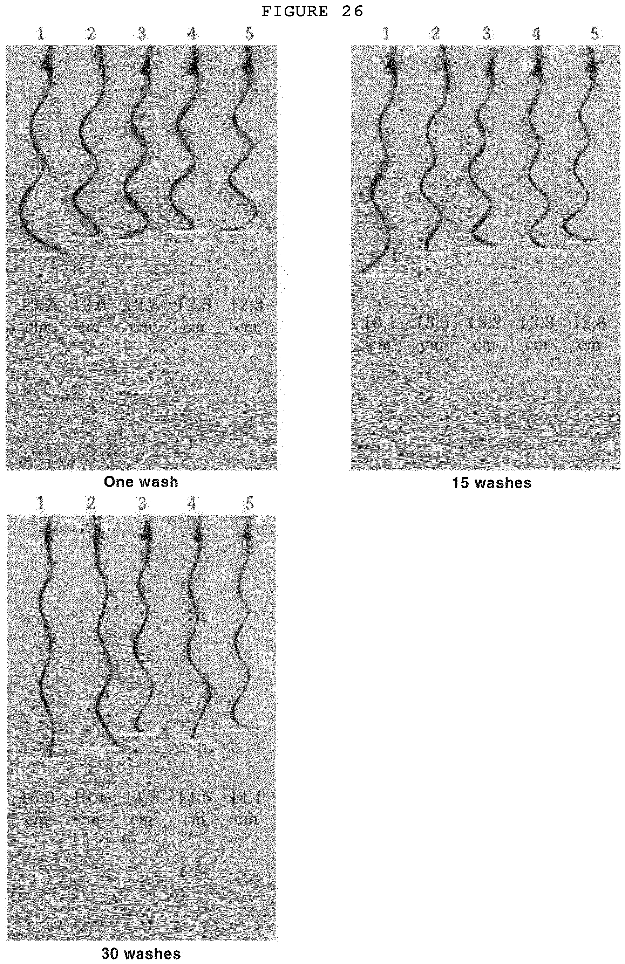

About 50 strands of natural black hair was fixed and cut to 17 cm in length and used in the experiment. The perm process was performed as follows. The dyed hair was fixed to 17 cm and the washing process was carried out. After washing, the dried hair bundle was reduced by treatment with the first agent (Hair 119 Clinic SuBoon Perm, Eson Chemical, Korea) at 37°C for 25 minutes. After the reduction process, to increase the reproducibility of wave efficiency, the first agent was removed by washing with distilled water. The washed hair was wound and fixed on a rod with a diameter of 8 mm. The hair was reoxidized by applying 20 ml of hydrogen peroxide, electrolyzed hydrogen water, and electrolyzed water were applied to the hair fixed on the rod. After the application was finished, the rod was removed. In order to measure the perm efficiency, the length was measured, and photos were taken after washing (FIG. 26) . Table 4 below shows the result of a change in the length of the hair according to the number of washes during the perm treatment of the dyed hair.

As shown in Table 4, in washing once after the perm treatment, washing once with electrolyzed water (column 4 and column 5) had better wave efficiency than washing once with hydrogen water (column 2 and column 3). In addition, in 30 washes, column 5 washed with electrolyzed water was the best, column 3 washed with hydrogen water had good wave efficiency almost the same as column 4 washed once with electrolyzed water once and others with distilled water. Therefore commercially, electrolyzed water is more advantageous in perm treatment.

**Table 4**

| Column | 1 | 2 | 3 | 4 | 5 |
|---|---|---|---|---|---|
| Number of washes | Washing with distilled water | Once with hydrogen water and others with distilled water | Washing with hydrogen water | Once with electrolyzed water and others with distilled water | Washing with electrolyzed water |
| One wash | 13.7 cm | 12.6 cm | 12.8 cm | 12.3 cm | 12.3 cm |
| 15 washes | 15.1 cm | 13.5 cm | 13.2 cm | 13.3 cm | 12.8 cm |
| 30 washes | 16.0 cm | 15.1 cm | 14.5 cm | 14.6 cm | 14.1 cm |

FIG. 5 shows the color change of the hair according to the number of washes when perm treatment of dyed hair. The color of the dyed hair was measured using a colorimeter spectrophotometer CM-2500d (Konica Minolta Sensing, Inc., Japan). L value represents thickness, a value represents red, and b value represents yellow. In FIG. 5, L value brightened according to the number of washes, and the brightness level was one wash > 15 washes > 30 washes. In 30 washes, the brightness level was washing in distilled water (column 1) > once with hydrogen water and others with distilled water (column 2) > once with electrolyzed water and others with distilled water > washing with hydrogen water (column 3) > washing with electrolyzed water (column 5) and washing with electrolyzed water showed the least of loss of dye. The red a value and yellow b value were not significantly different from each other in 15 and 30 washes.

**Table 5**

| **One wash** | **1** | **2** | **3** | **4** | **5** |
|---|---|---|---|---|---|
| L | 20.84 | 10.27 | 10.29 | 8.80 | 8.59 |
| a | 42.18 | 43.90 | 42.37 | 43.12 | 41.81 |
| b | 13.21 | 11.35 | 10.23 | 10.89 | 12.01 |

| **15 washes** | **1** | **2** | **3** | **4** | **5** |
|---|---|---|---|---|---|
| L | 30.71 | 29.64 | 26.59 | 24.46 | 20.15 |
| a | 49.39 | 50.60 | 50.12 | 49.30 | 48.35 |
| b | 16.91 | 16.30 | 16.25 | 15.84 | 15.45 |

| **30 washes** | **1** | **2** | **3** | **4** | **5** |
|---|---|---|---|---|---|
| L | 38.48 | 34.56 | 30.16 | 32.16 | 24.05 |
| a | 50.23 | 51.16 | 51.07 | 50.12 | 50.05 |
| b | 17.05 | 16.58 | 16.25 | 16.41 | 16.19 |

### 12. Washing Using Electrolyzed Water to Prevent Decreasing of Tensile Strength of Bleached Hair During Manufacturing Bleached Hair

Consumers desire blonde or blonde-like hair in some cases by bleaching their hair according to their aesthetic desire. At this time, the hair tissue is destroyed by the action of the bleaching agent and the tensile strength of the hair is lowered and the hair may break easily along with damage to the hair. Usually, to show blonde color, bleaching is performed about 3 times, but hair that has been bleached 3 times may break easily. As a way to complement this, an investigation has been conducted as to how to improve the decrease in tensile strength of the hair due to bleaching by using a hair care method that bleaches hair with electrolyzed water and hydrogen water and washes it.

### Measurement of Tensile Strength

The tensile strength was measured to find out the effect of electrolyzed water and hydrogen water on the tensile strength of the hair during the bleaching process. The bleaching agent used in the experiment was a mixture of multi blonde lightening powder (Wella, USA) and Welloxon perfect cream developer (Wella, USA) at a ratio of 1:2. The hair used for the experiment was washed once to remove foreign substances and contaminants, dried, and then used. The electrolyzed water group applied a bleaching agent to the entire washed hair, left it at 37°C for 20 minutes and washed it with electrolyzed water, which was bleached once. The distilled water group applied a bleaching agent to the entire washed hair, left it at 37°C for 20 minutes and washed it with distilled water, which was bleached once. The hydrogen water group bleached in the same way as the electrolyzed water. In this way, bleaching was repeated for the second and third times to make hair bleached three times in total and tensile strength was measured.

Tensile strength was measured according to the Korean Industrial Standard Tensile Strength Test Method (KS K ISO 5079:2007), in which 20 strands of bleached hair are randomly selected and single fibers are measured with a universal material strength tester (INSTRON 4465, INSTRON, USA).

The results of tensile strength measurement are as follows (FIG. 6). Natural hair showed a tensile strength of 164 gf/strand. In the case of bleaching with distilled water and then washing, the tensile strengths of 148 gf/strand, 126 gf/strand, and 98 gf/strand, respectively, were shown at first, second, and third bleaching, and in the case of bleaching with electrolyzed water and washing, the tensile strengths of 154 gf/strand, 138 gf/strand, and 122 gf/strand, respectively, were shown at first, second, and third bleaching. In the case of bleaching with hydrogen water and washing, the tensile strengths of 152 gf/strand, 133 gf/strand, 112 gf/strand, respectively, were shown at first, second, and third bleaching.

Based on 3 times of bleaching, distilled water bleached hair was damaged by 40.24%, electrolyzed water bleached hair was damaged by 25.60%, and hydrogen water bleached hair was damaged by 32.70%. Therefore, electrolyzed water prevented 14.64% damage than distilled water, and hydrogen water prevented 7.54% damage.

In addition, in the 3 types of bleached hair, in the visual evaluation, the distilled water group had split ends of the hair, and the overall hair surface was rough. Electrolyzed water group and hydrogen water group had no split ends, and their hair looked relatively neat and smooth, but the electrolyzed water group showed a better effect.

**Table 6**

| | Distilled water | Electrolyzed water | Hydrogen water |
|---|---|---|---|
| Natural hair | 164 gf/strand | | |
| Bleaching once | 148 gf/strand | 154 gf/strand | 152 gf/strand |
| Bleaching 2 times | 126 gf/strand | 138 gf/strand | 133 gf/strand |
| Bleaching 3 times | 98 gf/strand | 122 gf/strand | 112 gf/strand |

### 12. Washing Method Using Electrolyzed Water as a Method to Reduce Dye Leakage When Washing Dyed Hair

Hair dyeing is very often to dye white hair, which gives a dye to white hair, and to show color to virgin hair. At this time, as the hair is washed after dyeing, the dye is eluted from the dyed hair, and in most cases, the color of the dyed hair is usually faded. In most cases, the desired intrinsic color of dyed hair fades, causing aesthetic problems. Therefore, we devised a method of treating electrolyzed water as a method to reduce dye elution of dyed hair during washing after hair dyeing.

### Color Measurement of Dyed Hair

The dyes used in the experiment were 10/45 (red), 10/88 (blue) (Wella, USA) and 8G (dark brown) (AmorePacific, Republic of Korea) as hair dyes, and Welloxon Perfect Cream Developer (Wella, USA) as an oxidation agent in a 1:1 ratio was used.

The hair used in the red and blue dyeing experiment was washed once to remove foreign substances and contaminants, dried, and then treated with the bleaching agent described in "Measurement of Tensile Strength" for 15 minutes and then the washing process repeated twice and used. In the distilled water group, red, blue, and dark brown dyes were applied to the entire hair after washing, and then left at 37°C for 20 minutes and washed with distilled water, the electrolyzed water group was washed with electrolyzed water, and the hydrogen water group was washed with hydrogen water. In order to observe the color fading phenomenon due to washing, 14 washes were performed, and at the 1^{st} and 14th washes, observation and taking photos were performed using a colorimeter spectrophotometer CM-2500d (Konica Minolta Sensing, Inc., Japan).

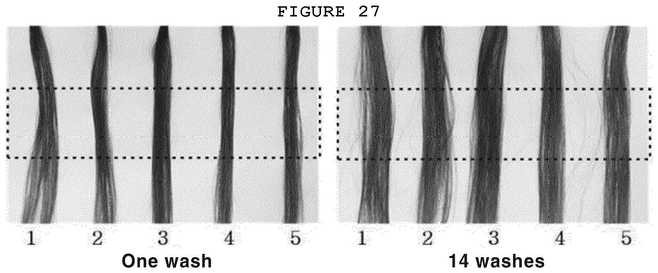

Column 1 in Tables 7 to 9 and FIGs. 27 to 29 show the hair of which all washes were performed with distilled water. Column 2 shows the hair that was washed with electrolyzed water only once in the initial stage and then washed with distilled water the other 13 times, and column 3 shows the hair that has been washed with electrolyzed water. Column 4 shows the hair that was washed with hydrogen water only once in the initial stage and then washed with distilled water the other 13 times, and column 5 shows the hair of which all washes were performed with hydrogen. Color measurement was performed by measuring the color in the dotted area in the middle of the hair. The color was measured using a colorimeter spectrophotometer CM-2500d (Konica Minolta Sensing, Inc., Japan).

There was no significant change in color saturation (a, b) in dark brown dyeing, but there was a difference in color darkness (L) of the color. In one wash out of the five groups in Table 5, compared to the distilled water wash (column 1), the electrolyzed water wash (column 3) and electrolyzed water one wash (column 2) causes 58.78% and 57.77%, respectively, and the hydrogen water one wash (column 4) and the hydrogen wash (column 5) caused 50.71% and 50.62%, respectively, less dark brown dye to escape (Table 7 and FIG. 27).

In addition, in 14 washes, the electrolyzed water wash (column 3) showed the highest result among 5 groups, which means that the electrolyzed water wash causes 34.39% less dark brown hair dye to escape compared to distilled water wash (column 1). Next, compared to the distilled water wash, the hydrogen water wash (column 5) caused 29.94%, the electrolyzed water one wash (column 2) 27.60%, and the hydrogen water wash (column 4) 23.50% less dark brown dye to escape (Table 7 and FIG. 27).

There was a difference in red color saturation (a) and in color darkness (L) in red dyeing. In terms of the red hair dye standard in one wash out of the five groups, compared to the distilled water wash (column 1), the electrolyzed water wash (column 3) and electrolyzed water one wash (column 2) causes 43.07% and 39.53%, respectively, and the hydrogen wash (column 5) and the hydrogen water one wash (column 4) caused 39.40% and 36.86 %, respectively, less color to escape. In addition, it can be seen that the stronger the red saturation (a), the darker the darkness (L).

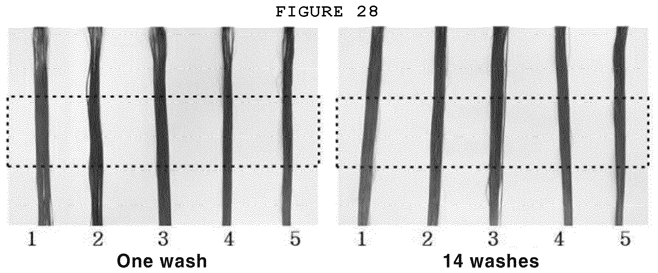

In addition, in 14 washes, the electrolyzed water wash showed the highest result, which means that the electrolyzed water wash (column 3) causes 52.86% less red hair dye to escape compared to distilled water wash (column 1). Next, compared to the distilled water wash, the hydrogen water wash (column 5) caused 44.23%, the electrolyzed water one wash (column 2) 41.69%, and the hydrogen water one wash (column 4) 37.02% less red dye to escape. In addition, it can be seen that the stronger the red saturation (a), the darker the darkness (L) (Table 8 and FIG. 28) .

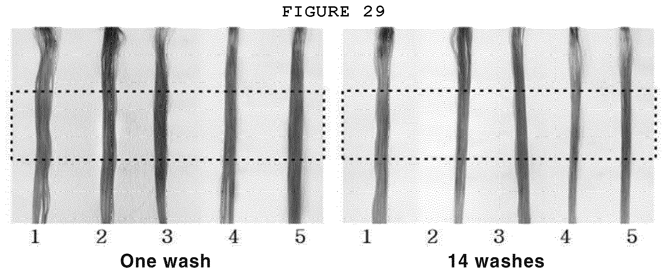

There was a difference in blue color saturation (a) and in color darkness (L) in blue dyeing. In terms of the blue hair dye standard in one wash out of the five groups, compared to the distilled water wash (column 1), the electrolyzed water wash (column 3) and electrolyzed water one wash (column 2) causes 35.81% and 33.95%, respectively, and the hydrogen wash (column 5) and the hydrogen water one wash (column 4) caused 11.16% and 7.006 %, respectively, less color to escape. In addition, it can be seen that the stronger the red saturation (a), the darker the darkness (L) (Table 9 and FIG. 29).

In addition, in 14 washes, the electrolyzed water wash (column 3) showed the highest result, which means that the electrolyzed water wash causes 65.71% less blue hair dye to escape compared to distilled water wash (column 1). Next, compared to the distilled water wash, the hydrogen water wash (column 5) caused 51.36%, the hydrogen water one wash (column 4) 22.58%, and the electrolyzed water one wash (column 2) 19.16% less blue dye to escape. In addition, it can be seen that the stronger the blue saturation (b), the darker the darkness (L) (Table 9 and FIG. 29).

**Table 7**

| **One wash** | **1** | **2** | **3** | **4** | **5** |
|---|---|---|---|---|---|
| L | 20.84 | 8.80 | 8.59 | 10.27 | 10.29 |
| a | 42.18 | 43.12 | 41.81 | 43.90 | 42.37 |
| b | 13.21 | 10.89 | 12.01 | 11.35 | 10.23 |

| **14 washes** | **1** | **2** | **3** | **4** | **5** |
|---|---|---|---|---|---|
| L | 30.71 | 24.46 | 20.15 | 29.64 | 26.59 |
| a | 49.39 | 49.30 | 48.35 | 50.60 | 50.12 |
| b | 16.91 | 15.40 | 16.21 | 16.15 | 16.70 |

**Table 8**

| **One wash** | **1** | **2** | **3** | **4** | **5** |
|---|---|---|---|---|---|
| L | 30.18 | 16.53 | 15.39 | 20.18 | 17.81 |
| a | 78.91 | 110.1 | 112.9 | 108.0 | 110.0 |
| b | 5.58 | 4.16 | 4.27 | 6.57 | 5.28 |

| **14 washes** | **1** | **2** | **3** | **4** | **5** |
|---|---|---|---|---|---|
| L | 32.43 | 23.48 | 21.39 | 24.81 | 22.72 |
| a | 70.72 | 100.2 | 108.1 | 96.90 | 102.0 |
| b | 10.40 | 11.08 | 9.28 | 10.76 | 10.21 |

**Table 9**

| **One wash** | **1** | **2** | **3** | **4** | **5** |
|---|---|---|---|---|---|
| L | 35.81 | 22.18 | 22.82 | 29.64 | 25.18 |
| a | 8.38 | 7.91 | 6.98 | 9.71 | 7.54 |
| b | 2.15 | 1.42 | 1.38 | 2.00 | 1. 91 |

| **14 washes** | **1** | **2** | **3** | **4** | **5** |
|---|---|---|---|---|---|
| L | 45.25 | 40.99 | 37.68 | 42.52 | 39.11 |
| a | 8.19 | 8.61 | 8.93 | 8.70 | 9.56 |
| b | 12.89 | 10.42 | 4.42 | 9.98 | 6.27 |

### 14. Difference between Hydrogen Water and Electrolyzed Water depending on Scale of Use

On a laboratory scale, electrolyzed water is more advantageous to prepare electrolyzed water or hydrogen water and use them in experiments. However, in the field, a large amount of electrolyzed water is required for perm treatment, bleaching, and wash after dyeing, etc. However, due to the mechanical characteristics, it is easy to obtain a large amount of electrolyzed water, which has a stronger effect, than hydrogen water. In other words, electrolyzed water is significantly more advantageous than hydrogen water because it can be quickly prepared in large quantities in the field of beauty salons. In addition, hydrogen water generator is more difficult to maintain than electrolyzed water generator. In other words, hydrogen water is difficult to obtain in large quantities, and it has the disadvantage that it is difficult to show the effect in perm, dyeing, and bleaching as much as electrolyzed water, and this can be clearly seen through the experiment below.

### Measurement of Cystine Generation Capacity in Hydrogen Water and Electrolyzed Water

Cysteine is unstable in air and has the property of combining with each other to form cystine (Hirs, C. W. (1967). Performic acid oxidation. In Methods in enzymology (Vol. 11, pp. 197-199). Academic Press.). In addition, cysteine precipitates insoluble in water. Through such properties, the amount of prepared cystine was measured by adding electrolysis hydrogen water and electrolyzed water with different open times. For preparation of the used electrolyzed hydrogen water and electrolyzed water, a 005% NaCl solution prepared by adding NaCl to distilled water was used to reduce experimental errors.

400 ml of raw material water was put into a hydrogen water generator (Grentech, Korea) with an output of 24W (12V, 2A) and hydrogen water generation process was performed for 20 minutes to obtain hydrogen water. The raw material water was put into an electrolyzed water generator with an output of 27W (9V, 3A) (Ecowell, Korea) and electrolyzed water generation process was performed for 20 minutes to obtain electrolyzed water, which was used as electrolyzed water.

After putting 0.75g of cysteine into each of ten 15 ml test tubes, generation of electrolyzed hydrogen water and electrolyzed water was started. From the start of generation, 15 ml of hydrogen water and electrolyzed water were injected into 10 test tubes containing cysteine every 2 minutes and reacted for 30 minutes, respectively. Since the cysteine dissolved in the cysteine solution is oxidized even at oxygen concentration in the air, the supernatant containing unreacted cysteine was removed by centrifugation (2000 x G, 5 minutes) after the reaction time was elapsed to prevent further reactions over time.

After the precipitated cystine was suspended by adding 3 ml of distilled water, turbidity was measured at 660 nm, and the amount of cystine preparation was calculated using a standard. The above experiment was repeated three times and the mean and standard error were calculated through IBM SPSS statistics for Windows, version 20.0 (IBM, USA).

The pH of electrolyzed hydrogen water and electrolyzed water prepared by electrolysis before the experiment was 6.8, and there was no difference before and after electrolysis.

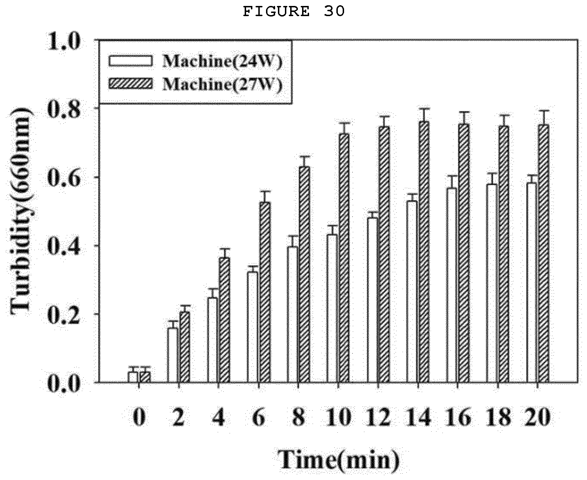

The turbidity of the precipitate generated by adding electrolyzed hydrogen water and electrolyzed water to cysteine is illustrated in FIG. 30. Immediately after the generation of electrolyzed hydrogen water and electrolyzed water, at 2 minute-intervals from immediately after generation, the resulting precipitate was measured by adding it to cysteine. As a result of measuring the concentration of the product with turbidity, the concentrations of the hydrogen water (24W apparatus) were 0.031, 0.158, 0.247, 0.322, 0.396, 0.431, 0.481, 0.53, 0.568, 0.579, and 0.583. Those of electrolyzed water (27 W apparatus) were 0.031, 0.205, 0.363, 0.526, 0.63, 0.726, 0.746, 0.762, 0.754, 0.748, and 0.751. As a result of adding electrolyzed hydrogen water to 0.75g of cysteine in this way, the resulting precipitate was calculated as weight of 0.0006g, 0.0029g, 0.0045g, 0.0059g, 0.0072g, 0.0079g, 0.0088g, 0.0097g, 0.0104g, 0.0106g, and 0.0107g, respectively, and the precipitate generated by adding electrolyzed water was calculated as weight of 0.0006g, 0.0038g, 0.0066g, 0.0096g, 0.0115g, 0.0133g, 0.0136g, 0.0139g, 0.0138g, 0.0137g, 0.0137g, respectively.

As such, it was found that the ability to promote cystine conversion from cysteine in the electrolyzed hydrogen water hardly increased from 16 minutes of the generation time. It was found that the ability to promote cystine conversion from cysteine in electrolyzed water hardly increased from 10 minutes. At 10 minutes from the start of cystine generation, cystine generation was 0.0079g for hydrogen water and 0.0133g for electrolyzed water, indicating that the hydrogen water showed 59% of cystine generation of electrolyzed water. In addition, at 16 minutes, hydrogen water showed 0.0104g and electrolyzed water 0.0138g of cystine generation, so hydrogen water showed 78% cystine generation of that of electrolyzed water. There is an 11% difference in output between the electrolyzed water generator with an output of 24W (12V, 2A) and the hydrogen water generator with an output of 27W (9V, 3A). Therefore, it can be seen that the hydrogen water generator has a lower cystine generation ability by 30% at 10 minutes and 11% at 16 minutes than the electrolyzed water generator.

Therefore, the electrolyzed water generator has a faster cystine generation rate and a higher yield rate than the hydrogen water generator. In addition, hydrogen water generator is difficult to expand due to the installation of a valve whereas electrolyzed water generator does not require a valve. Electrolyzed water generator is more advantageous for commercial because there is a need of scale-up to wash hair for several purpose (dandruff removal, wave maintenance, dyeing, and tensile strength).

### Industrial Applicability

According to a method of forming a hair perm using electrolyzed water of the present invention, wherein the method uses the electrolyzed water several times or for a specific time or longer, the method has been found to have the effects enabling to repeat treatments due to very little damage to hair, to perm damaged hair and blonde hair compared to the existing method, to have an oxidizing power greater than hydrogen peroxide, to cause less damage to hair, to suppress dandruff, and to significantly reduce dye elution after perm. Therefore, the method of forming a hair perm, using electrolyzed water of the present invention several times or for a specific time or longer, can be usefully used as a method of effectively form a hair perm without harm to the human body in the field of forming a hair perm. In addition, in the case of washing dying/perm-treated hair, dyed hair, and bleached hair with electrolyzed water, compared to washing with distilled water, it has the advantage that the tensile strength of the hair is maintained and there is less loss of dye from the dyed hair.

## Claims

1. A method of forming a hair perm, the method comprising:
(a) treating hair with a reduction agent and then fixing the hair; and
(b) treating the fixed hair with electrolyzed water twice or more.

2. The method of claim 1, wherein the hair in step (a) is one or more hairs selected from the group consisting of natural black hair, blonde hair, bleached hair, curly hair, and brown hair.

3. The method of claim 1, wherein the electrolyzed water in step (b) is electrolyzed hydrogen water or electrolyzed water.

4. The method of claim 3, wherein the electrolyzed hydrogen water is prepared in a hydrogen water generator equipped with a cation exchange membrane.

5. The method of claim 3, wherein the electrolyzed water is prepared with a process of electrolyzing water without separating an anode and a cathode.

6. The method of claim 1, wherein the electrolyzed water in step (b) is prepared by an electrolyzed water generator with an output power of 11W or more.

7. The method of claim 1, wherein the electrolyzed water in step (b) is used within 30 minutes from being prepared.

8. The method of claim 1, wherein the electrolyzed water in step (b) has a temperature of 10°C to 90°C.

9. The method of claim 1, wherein the electrolyzed water in step (b) has a pH of 0.7 to 11.5.

10. The method of claim 1, wherein the perm is a permanent wave or a straight perm.

11. Electrolyzed water for forming a hair perm.

12. The water of claim 11, wherein the electrolyzed water is electrolyzed hydrogen water or electrolyzed water.

13. The water of claim 12, wherein the electrolyzed hydrogen water is prepared in a hydrogen water generator equipped with a cation exchange membrane.

14. The water of claim 12, wherein the electrolyzed water is prepared with a process of electrolyzing water without separating an anode and a cathode.

15. The water of claim 11, wherein the electrolyzed water is prepared by an electrolyzed water generator with an output of 11W or more.

16. The water of claim 11, wherein the electrolyzed water has a temperature of 10°C to 90°C.

17. The water of claim 11, wherein the electrolyzed water has a pH of 0.7 to 11.5.

18. A composition for washing hair, including electrolyzed water.

19. The composition of claim 18, wherein the electrolyzed water is electrolyzed water or hydrogen water.

20. The composition of claim 18, wherein the hair is dye/perm-treated hair or dye-treated hair.

21. A method of washing hair, the method comprising the step of treating hair with electrolyzed water.

22. The method of claim 21, wherein the electrolyzed water is electrolyzed water or hydrogen water.

23. The method of claim 21, wherein the is dye/perm-treated hair or dye-treated hair.
